(19) Europäisches Patentamt / European Patent Office / Office européen des brevets

(11) **EP 4 431 551 A1**

(12) **EUROPEAN PATENT APPLICATION**
published in accordance with Art. 153(4) EPC


(43) Date of publication:
**18.09.2024 Bulletin 2024/38**

(21) Application number: **21963541.4**

(22) Date of filing: **10.11.2021**

(51) International Patent Classification (IPC):
***C08J 3/24*** *(2006.01)* ***C08J 3/075*** *(2006.01)*
***C08L 5/08*** *(2006.01)* ***C08K 5/17*** *(2006.01)*
***C08B 37/08*** *(2006.01)*

(52) Cooperative Patent Classification (CPC):
**C08B 37/003; C08J 3/075; C08J 3/24; C08K 5/17; C08L 5/08**

(86) International application number:
**PCT/CN2021/129743**

(87) International publication number:
**WO 2023/082084 (19.05.2023 Gazette 2023/20)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**



(71) Applicant: **Imeik Technology Development Co., Ltd.**
**Beijing 100022 (CN)**

(72) Inventors:
- **LI, Ruizhi**
  **Beijing 100022 (CN)**
- **GU, Shiwei**
  **Beijing 100022 (CN)**
- **ZHANG, Kun**
  **Beijing 100022 (CN)**

(74) Representative: **Studio Torta S.p.A.**
**Via Viotti, 9**
**10121 Torino (IT)**


(54) **GEL MATERIAL, PREPARATION METHOD THEREFOR, AND USE THEREOF**


(57) A gel material, a preparation method therefor, and a use thereof. The gel material is obtained by cross-linking endogenous polyamine with Hyaluronic Acid (HA), and the endogenous polyamine includes spermine and/or spermidine. The cross-linking between the endogenous polyamine and the HA includes two-site cross-linking, three-site cross-linking, or four-site cross-linking. The various properties of the gel and degradation and release rates of the polyamine are affected by controlling reactive cross-linking sites. After the gel is subjected to moist heat sterilization, a loss rate of elastic modulus is low, such that the rheological property of the gel before sterilization may be effectively maintained, thereby greatly improving the thermal stability of the HA gel, and also improving the ease of use of the gel in fields such as soft tissue filling, soft tissue repair, medical cosmetology, etc.


Fig. 1

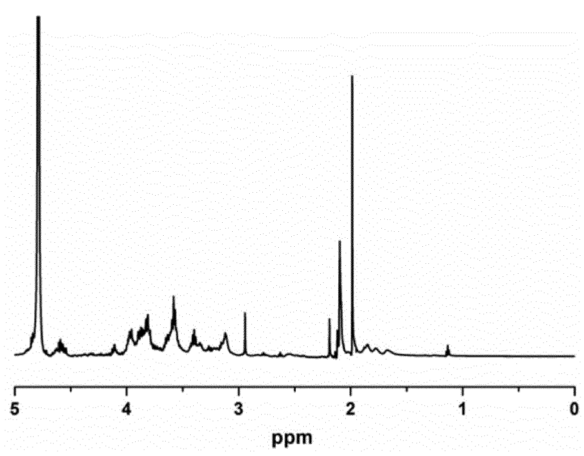


EP 4 431 551 A1

## Description

### Technical Field

The present disclosure relates to the technical field of biomedical materials, and in particular, to a gel material, a preparation method therefor, and a use thereof.

### Background

Hyaluronic acid (HA) is also referred to as sodium hyaluronate, and is a glycosaminoglycan composed by disaccharide units of D-glucuronic acid and N-Acetylglucosamine, linked together via alternating β-1,4 and β-1,3 glycosidic bonds. The HA is widely used in cosmetics or ophthalmic surgery, and may also be used to correct wrinkles and some soft tissue defects as a soft tissue filler. The HA is an endogenous substance with good biocompatibility and certain biological activity. However, exogenous HA will be degraded by hyaluronidase in the body to shorten residence time in the body, resulting in shortening of a treatment effect, and the treatment effect can only be achieved through a plurality of injections. In order to prevent the HA from being degraded by the hyaluronidase, HA need to be cross-linked through a chemical cross-linking agent to form a space network structure, and the degradation of the hyaluronidase on the HA is prevented through a dense rigid network structure, such that the residence time of the exogenous HA in the body is prolonged, thereby achieving a good treatment effect while guaranteeing the biocompatibility.

Cross-linking agents for cross-linked HA currently available in the market are mainly classified into two categories; one category is double epoxy cross-linking agents, which mainly include 1,4-butanediol diglycidyl ether (BDDE); and the other category is unsaturated sulfone cross-linking agents, which mainly include divinyl sulphone (DVS). The two categories of cross-linking agents have a similar mechanism, which takes an alkaline catalyst as a prerequisite to catalyze hydroxyl (-OH) in the HA to undergo an addition reaction with the cross-linking agent, so as to complete cross-linking; and BDDE undergoes an open-loop addition reaction with the -OH in the HA to complete cross-linking, and DVS undergoes a Michael addition reaction with the -OH in the HA to complete cross-linking. However, these two categories of cross-linking agents both have high biological toxicity, and at the same time, unreacted monomers or by-products of cross-linking reactions are also potentially carcinogenic. Since HA gel is a long-term implantable medical device product, there is also a lack of long-term data tracking with sufficient sample sizes on a clinical side to demonstrate the biosafety of the product. In terms of current market requirements, prolonging the residence time of the HA gel in the body and increasing the viscoelastic properties of the HA gel are major trends in the industry today. An existing method that can effectively prolong the residence time of the HA gel in the body is to increase a cross-linking degree, and the increasing of the cross-linking degree can also enhance the viscoelastic properties. However, when traditional BDDE or DVS cross-linking agents are used, increasing addition amounts of the cross-linking agents increases concerns about the safety of the product.

On this premise, the choice of non-toxic cross-linking agents for the HA gel will also be more sought after by more HA gel manufacturers around the world. At present, non-toxic amino acid cross-linking agents have been used in the HA gel. A Chinese Patent CN10105713211 disclosed cross-linked HA gel prepared using 4-(4,6-dimethoxytriazine-2-yl)-4-methylmorpholine hydrochloride as a condensation agent, and using lysine and derivatives thereof or arginine and derivatives thereof as a cross-linking agent. Chinese Patents CN106188609, CN106188584, and CN111732741 disclosed cross-linked HA gel prepared using carbodiimide such as 1-(3-dimethylaminopropyl)-3-ethylcarbodiimide hydrochloride as a condensation agent, using succinimide such as N-hydroxysuccinimide as a catalyst, and using arginine and derivatives thereof or lysine and derivatives thereof (containing polylysine) as a cross-linking agent. The above patents all disclosed the cross-linked sodium hyaluronate gel that was prepared by using diamino amino acids such as the lysine or the arginine as the cross-linking agent. Using the lysine or arginine cross-linking agent as an example, the cross-linked HA hydrogel shows a large performance difference during moist heat sterilization. CN106188609, CN106188584, and CN111732741 did not reflect that the cross-linked HA could be subjected to moist heat sterilization for 15 minutes at 121°C. CN10105713211 disclosed lysine cross-linked HA gel that could be subjected to moist heat sterilization. However, information disclosed by the patent shows that a minimum degradation rate of the gel after moist heat sterilization has exceeded 40%, indicating that the moist heat sterilization performance of the gel is poor. US9907739B2 disclosed a cross-linking reaction between spermine or spermidine and the HA. However, when detection is performed according to the method of the present disclosure, the gel disclosed in US9907739B2 is only two-site cross-linked, and does not involve multi-site cross-linking, i.e., a cross-linked site is only an amino group in the spermine or spermidine, an imino group does not participate in a reaction, and the loss rate of elastic modulus of the gel after moist heat sterilization is up to 23%, also indicating that the moist heat sterilization performance of the gel is poor. In addition, Xiang Mei Yan et. al. published an article (Journal of Biomaterials Applications, 2011, 27(2):179-186) in which HA hydrogel cross-linked with hexamethylenediamine was disclosed, but a decreased value of the elastic modulus of the gel after sterilization also exceeded 20%. The main reason for this is the presence of only two amino cross-linking sites

in such diamine molecules such as the lysine, the arginine, and the hexamethylenediamine. When cross-linking occurs, the cross-linking sites form an amide bond with carboxyl in the HA so as to complete cross-linking. However, since there are fewer cross-linking sites in the molecules, a formed cross-linking network structure cannot provide adequate thermal stability protection for an HA main chain, in combination with the poor thermal stability of the amide bond itself, which is reflected in the gel as a high degradation rate and poor thermal stability after moist heat sterilization.

Endogenous polyamine mostly refers to polyamine that can be produced during synthetic or metabolic processes in a human body environment, and the main endogenous polyamine includes the spermine, the spermidine, and putrescine. Literature (Madeo et. al., Science 359, 410, 2018) reported that the endogenous polyamine, such as the spermidine, had specific physiological roles, including, but not limited to, circadian rhythm regulation, hypertension improvement, cardiovascular protection, Alzheimer's disease prevention, immunity enhancement, cancer resistance, and even aging prevention. The physiologically active effects of the spermidine are manifested in the following aspects: 1) kidney: tension reduction, and aging prevention; 2) heart: blood pressure lowering, and arteriosclerosis prevention; 3) brain: memory decline prevention, Alzheimer's disease resistance, neuroprotective effect; 4) bone: prevention of bone loss affected by ovariectomy; 5) skeletal muscle: increasing of the temperature of aged muscle, and muscle disease prevention; 6) whole organism: living organism lifespan prolonging; 7) immune system: improvement of immune activity after vaccination, improvement of cancer directed immunity, and fatal sepsis prevention; 8) liver: prevention of liver fibrosis and canceration, etc. Main mechanisms for the physiological activity of the spermidine are that, the spermidine is polycationic ($-NH^{3+}$) fatty amine that exists in a multiprotonated form under physiological pH conditions and is highly biologically active, and nucleic acids containing acidic residues, phospholipids, acidic proteins, pectic polysaccharides containing carboxylates or sulfates, as well as neurotransmitters and hormones (e.g., dopamine, adrenaline, serotonin, thyroid hormones, etc.) with a similar structure, are all possible targets for spermidine binding. In terms of binding to the nucleic acids, most of the polyamine exists in the form of polyamine-RNA complexes within cells. The main role of the spermidine is related to structural changes and translation of RNA, for example, by affecting a secondary structure of mRNA, tRNA, and rRNA to influence various stages of protein synthesis. The spermidine can also form a stable bridge between a double helical DNA strand, thereby reducing the accessibility of ROS or other DNA damaging agents and protecting DNA from thermal denaturation and X-ray radiation. In terms of protein binding, the spermidine can bind to a large number of negatively charged proteins, changing the spatial conformation of the proteins, and thus affecting their physiological functions, for example, protein kinases/phosphatases (important links in a plurality of signaling pathways), enzymes participating in histone methylation and acetylation (affecting gene expression by changing epigenetics), ion channel receptors (e.g., AMPA, AMDA receptors), and the like.

At present, there are few reports on the use of the endogenous polyamine such as the spermidine as the cross-linking agent for the HA gel, and there are virtually no reports on the cross-linking reaction conditions of the endogenous polyamine with the HA and the properties of the cross-linked hydrogel, in view of which the present disclosure is proposed.

## Summary

The present disclosure is intended to provide a gel material, a preparation method therefor, and a use thereof. By means of the preparation method of the present disclosure, cross-linking sites of endogenous polyamine and HA may be controlled. By controlling cross-linking reaction sites, various performance parameters of the prepared cross-linked HA gel are affected, and a release rate of the endogenous polyamine such as spermidine during the degradation of the HA gel is controlled, so as to continuously achieving the physiologically active effects of the endogenous polyamine such as the spermidine.

The present disclosure provides the following technical solutions.

In one aspect, the present disclosure provides a gel material. The gel material is mainly obtained by cross-linking endogenous polyamine with HA, and the endogenous polyamine includes spermine (a tetraamino compound) and/or spermidine (a triamino compound). The cross-linking between the endogenous polyamine and the HA includes two-site cross-linking, three-site cross-linking, or four-site cross-linking. In particular, in the present disclosure, an active star network structure of multi-site cross-linking that is formed by performing three-site or four-site cross-linking on the endogenous polyamine and the HA is realized for the first time.

In the present disclosure, the spermine or spermidine of the endogenous polyamine is selected as a multi-site cross-linking agent to form a compact network structure, such that the thermal stability of the amide bond cross-linked HA gel formed in such way is improved. The reason why endogenous diamine such as putrescine is not selected in the present disclosure lies in that, the diamine such as the putrescine has high toxicity, and the cross-linking sites are the same as those of amino acids such as lysine and arginine, which do not have the possibility of realizing multi-site cross-linking.

In an embodiment, the present disclosure provides a gel material. The gel material is obtained by cross-linking the endogenous polyamine with the HA, and the endogenous polyamine includes the spermine and/or the spermidine. The cross-linking between the endogenous polyamine and the HA includes the two-site cross-linking, the three-site

cross-linking, or the four-site cross-linking.

Further, the proportion of amino residues in the gel obtained through the two-site cross-linking is lower than 20%; preferably, the proportion of the amino residues in the gel obtained through the two-site cross-linking is lower than 15%; and more preferably, the proportion of the amino residues in the gel obtained through the two-site cross-linking is lower than 10%.

Further, the proportions of amino residues and imino residues in the gel obtained through the three-site cross-linking or four-site cross-linking are all lower than 20%.

Further, the proportion of the amino residues in the gel obtained through the three-site cross-linking or four-site cross-linking is lower than 15%; and preferably, the proportion of the amino residues in the gel obtained through the three-site cross-linking or four-site cross-linking is lower than 10%.

Further, the proportion of the imino residues in the gel obtained through the three-site cross-linking or four-site cross-linking is lower than 15%; and preferably, the proportion of the imino residues in the gel obtained throughthe three-site cross-linking or four-site cross-linking is lower than 10%.

Further, cross-linking reaction efficiency of the two-site cross-linking, three-site cross-linking, or four-site cross-linking in a cross-linking reaction between the endogenous polyamine and the HA is higher than 75%; preferably, the cross-linking reaction efficiency is higher than 80%; and more preferably, the cross-linking reaction efficiency is higher than 85%.

Further, a loss rate of elastic modulus (G' loss rate) of the gel obtained through cross-linking is lower than 22%; preferably, the loss rate of the elastic modulus (G' loss rate) is lower than 15%; and more preferably, the loss rate of the elastic modulus (G' loss rate) is lower than 10%.

In another aspect, the present disclosure provides a method for preparing a gel material. The method includes the following steps.

The pH of a mixed solution of HA and endogenous polyamine is regulated to 4.50-6.50, and an activating agent is added to cause the HA and the endogenous polyamine to undergo a two-site, three-site, or four-site cross-linking reaction, so as to obtain the gel material.

The endogenous polyamine includes spermine and/or spermidine.

The spermidine and the spermine have amino group and imino group; and the spermidine contains one imino group and two amino groups, and the spermine contains two amino groups and two imino groups. In the present disclosure, it is found that, by regulating the pH value of the mixed solution after the HA and the endogenous polyamine are dissolved, reaction sites of the spermine or spermidine and the HA are controlled, two-site cross-linked hydrogel and three-site or four-site cross-linked active star network gel may be obtained. During a reaction process, by regulating the pH of the solution, a reaction (i.e., a two-site reaction) may be performed at amino sites of the spermine or spermidine, or a co-reaction (i.e., a multi-site reaction) may be performed on the amino group and the imino group. The two-site cross-linking described in the present disclosure means that, when the HA is cross-linked with the spermine or spermidine, the amino group in the spermine or spermidine is the main reaction site, and in particular, the proportion of the amino residues in the gel obtained through the two-site cross-linking is lower than 20%. The three-site or four-site cross-linking described in the present disclosure means that, when the HA is cross-linked with the spermine or spermidine, the amino group and the imino group in the spermine or spermidine are used as the reaction sites together, and in particular, the proportions of amino residues and imino residues in the gel obtained through the three-site cross-linking or four-site cross-linking are all lower than 20%.

In the present disclosure, it is found that the reaction activity of the imino group may be improved by controlling the pH value of the soution between 5.00-5.49, so as to relatively stably realize the three-site or four-site reaction; and the reaction activity of the imino group may be provided by controlling the pH value of the soution between 4.50-4.99 or 5.50-6.50, so as to relatively stably make two sites participate in the reaction together, thereby preparing the active star network gel. Therefore, in the present disclosure, the HA hydrogel using the imino group as the cross-linking site is obtained for the first time, and it is found that the hydrogel with different cross-linking sites may achieve different gel properties at the same cross-linking degree.

In the present disclosure, the three-site or four-site reaction may be relatively stably realized by controlling the pH of the mixed solution between 5.00-5.49, and the pH includes, but is not limited to 5.00, 5.10, 5.20, 5.30, 5.40, or 5.49. The two-site cross-linking reaction may be relatively stably realized by controlling the pH of the mixed solution between 4.50-4.99 or 5.50-6.50, and the pH includes, but is not limited to 4.50, 4.60, 4.70, 4.80, 4.90, 4.99, 5.50, 5.60, 5.70, 5.80, 5.90, 6.00, 6.10, 6.20, 6.30, 6.40, or 6.50. In addition to the pH limited in the present disclosure, experimental results are not ideal. A pH value range of the present disclosure is a pH condition suitable for the method of the present disclosure that has been mapped out through extensive experiments.

The method of the present disclosure is a Spermidine/Spermine Multisite Active Reaction Technology (SMART). By means of the method of the present disclosure, the cross-linking sites of the endogenous polyamine and the HA may be controlled. By controlling the cross-linking reaction sites, various performance parameters of the cross-linked HA gel are affected. In addition, the release rate of the endogenous polyamine such as the spermidine during the degradation

of the HA gel may also be controlled, so as to continuously achieving the physiologically active effects of the endogenous polyamine such as the spermidine.

In an embodiment, the activating agent is added in the process of the two-site cross-linking reaction, three-site cross-linking reaction, or four-site cross-linking reaction.

Preferably, the activating agent includes one or more of water-soluble carbodiimide, phosphonium bromide salt formed by triphenylphosphine and bromide, carbonium salt, and 4-(4,6-dimethoxytriazine-2-yl)-4-methylmorpholine hydrochloride (DMTMM).

In an embodiment, the water-soluble carbodiimide activating agent includes 1-ethyl-3-(3-dimethylaminopropyl)carbodiimide (EDC), 1-(3-dimethylaminopropyl)-3-ethyl-carbodiimide, 1-cyclohexyl-3-(2-morpholinoethyl)carbodiimide, 1,3-bis[di(methoxymethyl)methyl]carbodiimide, or salts thereof, as well as a mixture of one or more of the above.

In an embodiment, the phosphonium bromide salt formed by the triphenylphosphine and the bromide includes a phosphonium salt formed by the triphenylphosphine and carbon tetrabromide, a phosphonium salt formed by the triphenylphosphine and N-bromosuccinimide, etc. The phosphonium bromide salt is obtained by a known method, and the required phosphonium salt is obtained by the triphenylphosphine and the bromide in dichloromethane.

In an embodiment, the carbonium salt includes a mixture of one or more of O-(7-azabenzotriazole-1-yl)-bis(dimethylamino)carbonium hexafluorophosphate (HATU), O-(benzotriazol-1-yl)-bis(dimethylamino)carbonium hexafluorophosphate (HBTU), O-(5-chlorobenzotriazol-1-yl)-bis(dimethylamino)carbonium hexafluorophosphate (HCTU), O-(benzotriazol-1-yl)-bis(dimethylamino)carbonium tetrafluoroborate (TBTU), O-(N-succinimidyl)-bis(dimethylamino)carbonium tetrafluoroborate (TSTU), and 2-(5-norborene-2,3-dicarboximido)-1,1,3,3-tetramethyluronium tetrafluoroborate (TNTU).

In the activating agent, when the water-soluble carbodiimide activating agent is used, the activating agent needs to be used in combination with an additive at the same time to improve the cross-linking reaction efficiency. Preferably, the additive includes one or more of N-hydroxysuccinimide (NHS), sulfonated N-hydroxysuccinimide (Sulfo-NHS), tert-butanol, and 1-hydroxybenzotriazole (HOBt). More preferably, an addition amount of the additive is 10-30% of the mass of the carbodiimide, including, but not limited to, 10%, 11 %, 12%, 13%, 14%, 15%, 16%, 17%, 18%, 19%, 20%, 21%, 22%, 23%, 24%, 25%, 26%, 27%, 28%, 29%, and 30%. The cross-linking reaction efficiency may reach about 70% through the combined use of the water-soluble carbodiimide and the additive.

In the activating agents of the phosphonium bromide salt formed by the triphenylphosphine and the bromide, the carbonium salt, and the 4-(4,6-dimethoxytriazine-2-yl)-4-methylmorpholine hydrochloride, the cross-linking efficiency of the carbonium salt is the highest, which is 80-95%. When a HATU catalyst is used, the cross-linking efficiency is up to 95%. The cross-linking efficiency of the phosphonium bromide salt and the DMTMM comes second, which is within a range of 70-85%.

In a preferred embodiment, the HATU of the carbonium salt is used as the reaction activating agent. A molecular formula structure of the HATU is shown in (1) below.

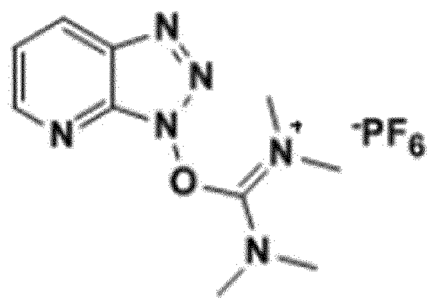

HATU (1).

A mechanism of the HATU participating in an amide condensation reaction is shown as follows.

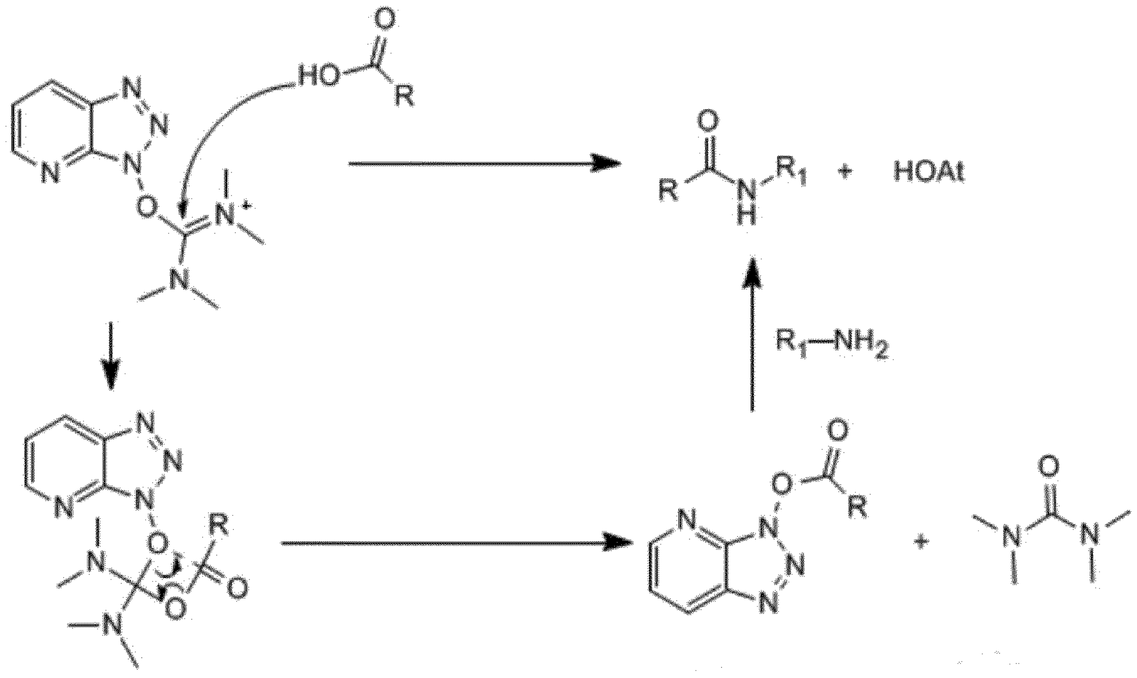


(2).

In the cross-linking reaction, when the two-site cross-linking is performed (the main reaction site of the spermine or spermidine is the amino group), the addition amount of the activating agent is 200-280% of the amount of substance of the endogenous polyamine, including, but not limited to, 210%, 220%, 230%, 240%, 250%, 260%, 270%, and 280%. When the three-site or four-site cross-linking is performed (the imino sites also participate in the reaction), the addition amount of the activating agent is 300-550% of the amount of substance of the endogenous polyamine, including, but not limited to, 320%, 350%, 370%, 390%, 400%, 420%, 450%, 470%, 500%, 520%, and 550%.

Preferably, when the endogenous polyamine is the spermine, the addition amount of the activating agent is 400-550% the amount of substance of the spermine, and when the endogenous polyamine is the spermidine, the addition amount of the activating agent is 300-400% the amount of substance of the spermidine.

The addition amount of the activating agent is related to the amino cross-linking sites of the cross-linking agent spermine or spermidine. When the activating agent is used, the activating agent of each molecule may activate one carboxyl group, and undergoes an amide coupling reaction with one amino group (or imino group).

In an embodiment, in the cross-linking reaction, when the endogenous polyamine is the spermine, an addition amount of the spermine is 0.3-35% of the mass of the HA, including, but not limited to, 0.4%, 0.5%, 0.8%, 1%, 3%, 5%, 8%, 10%, 11%, 12%, 13%, 14%, 15%, 16%, 17%, 18%, 19%, 20%, 21%, 22%, 23%, 24%, 25%, 26%, 27%, 28%, 29%, 30%, and 35%. When the endogenous polyamine is the spermidine, an addition amount of the spermidine is 0.5-40% of the mass of the HA, including, but not limited to, 0.5%, 0.8%, 1%, 3%, 5%, 8%, 10%, 11%, 12%, 13%, 14%, 15%, 16%, 17%, 18%, 19%, 20%, 21%, 22%, 23%, 24%, 25%, 26%, 27%, 28%, 29%, 30%, 34%, 38%, and 40%.

Further, a mass concentration of the HA in a mixed solution of a reaction system is 10-150 mg/mL, including, for example, but not limited to, 10, 20, 30, 40, 50, 60, 70, 80, 90, 100, 110, 120, 130, 140, or 150 mg/mL.

Further, in finally-obtained cross-linked HA hydrogel, the mass concentration of the HA is 1-50 mg/mL, including, but not limited to, 1, 3, 5, 10, 15, 20, 25, 30, 35, 40, 45, 50 mg/mL.

In an embodiment, the molecular weight of the HA is 100 KDa (kilodaltons) to 3000 KDa; and preferably, the HA is that prepared by a microbial fermentation method.

In an embodiment, in the cross-linking reaction, when the activating agent includes a carbonium salt and/or a phosphonium bromide salt formed by triphenylphosphine and bromide, the temperature of the cross-linking reaction is 10-60°C, and the time for the cross-linking reaction is 14-24 h.

When the activating agent includes water-soluble carbodiimide and/or 4-(4,6-dimethoxytriazine-2-yl)-4-methylmorpholine hydrochloride, the temperature of the cross-linking reaction is 40-60°C, and the time for the cross-linking reaction is 14-24 h.

During the reaction, the two-site cross-linked gel and the three-site or four-site cross-linked active star network gel may be controlled through different reaction temperatures and the ratio of the activating agent to the spermine or spermidine. Due to the impact of a steric hindrance effect on the imino group, the reaction activity of the imino group is

lower than that of the primary amino group, such that, in the two-site reaction in which only the primary amino group participates, only a low temperature and a low addition amount of the activating agent are required to complete the cross-linking reaction. However, when the imino group is required to participate in the reaction, in order to improve the reaction activity of the imino group, a reaction temperature needs to be increased, and reaction time is prolonged to improve the cross-linking reaction efficiency of the imino group.

The cross-linking reactions between the spermidine and the HA, and between the spermine and the HA are shown as follows. (3) is the cross-linking reaction (4-site star network) between the HA and the spermine. (4) is the cross-linking reaction (3-site star network) between the HA and the spermidine.

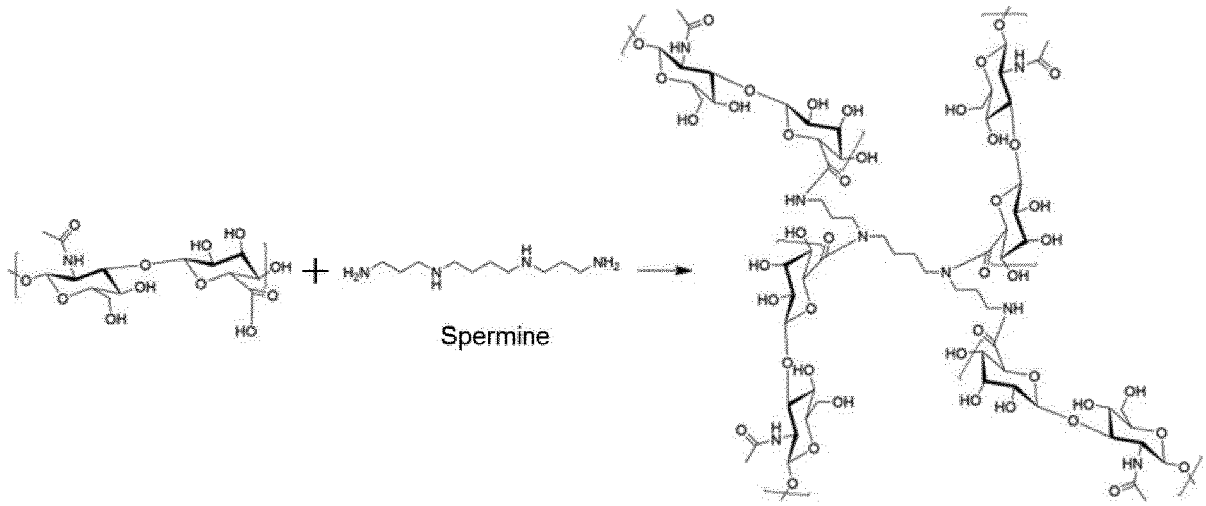


(3).

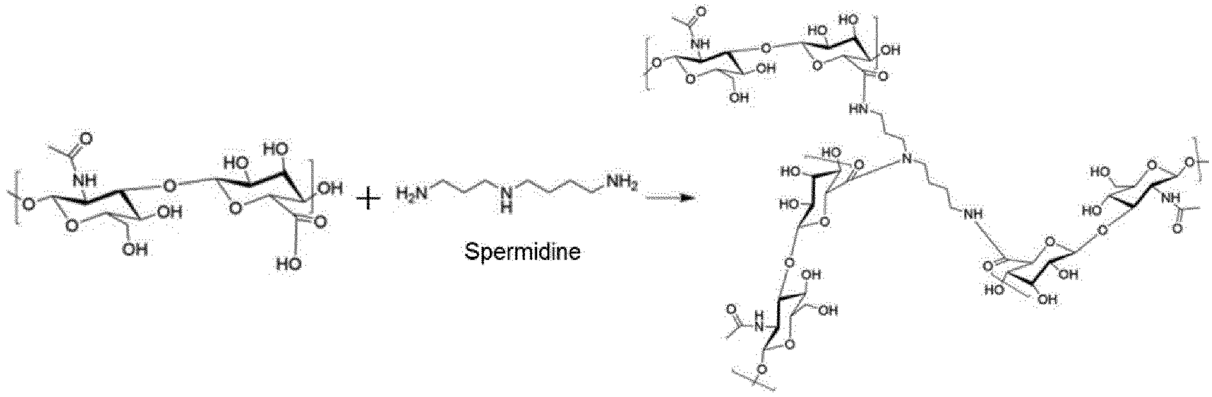


(4).

In the present disclosure, according to the HA gel prepared by using the SMART, different activities of the amino and imino groups in spermine or spermidine molecules are fully utilized. By controlling a reaction condition, e.g., one or more of the pH, the temperature, and the type and addition amount of the activating agent, the two-site cross-linked gel and the three-site or four-site cross-linked active star network gel may be respectively obtained.

In an embodiment, the gel material is further treated through elution by an eluent, crushing, and drying.

Preferably, the eluent is an organic solvent; more preferably, the organic solvent is soluble alcohol or soluble ketone; more preferably, the organic solvent is ethanol or acetone.

Further, a volume ratio of the gel to the eluent in the reaction system during crushing is 1:1-5.

Further, a particle size of the gel after crushing is 10-500 μm.

In an embodiment, the preparation method further includes performing re-dissolution and moist heat sterilization on the dried gel material.

Preferably, a solution for the re-dissolution is a phosphate buffer solution; and more preferably, a mass concentration of a phosphate buffer salt in the phosphate buffer solution is 5-40 mg/mL.

Further, the temperature of the moist heat sterilization is 120-130°C, and the time for the moist heat sterilization is preferably 15-45 min.

In a specific embodiment, the organic solvent is used to elute the gel, gel particles are crushed, and washing may be performed repeatedly for a plurality of times to remove the residual activating agent. The drying may use vacuum drying, and may remove the organic solvent. Dissolution efficiency of the residual activating agent during a plurality of cleaning processes may be guaranteed by crushing the gel to a low particle size.

In a specific embodiment, the gel particles after vacuum drying are re-dissolved with the phosphate buffer solution, filled in a prefilled syringe, and subjected to moist heat sterilization, so as to obtain the final product gel. The pH value of the phosphate buffer solution ranges from 6.8-7.6. The concentration of the final product HA gel is 1-35 mg/mL.

In a specific embodiment, the method includes the following steps.

(a) The HA and the endogenous polyamine are directly dissolved in water, and the pH value of the solution is regulated.
(b) The activating agent is added to complete the cross-linking reaction between the spermidine and/or spermine and the HA.
(c) The organic eluent is added, the gel particles are crushed, the residual activating agent is removed by performing washing for a plurality of times, and the organic solvent is removed through vacuum drying.
(d) The gel particles after vacuum drying are re-dissolved with the phosphate buffer solution, filled in a prefilled syringe, and subjected to moist heat sterilization, so as to obtain the final product gel.

After the HA gel prepared by the method of the present disclosure is subjected to moist heat sterilization, the loss rate of the elastic modulus is relatively low, which may be minimized to less than 10%, such that the rheological property of the gel before sterilization may be effectively maintained, thereby greatly improving the thermal stability of the HA gel.

In the present disclosure, while the polyamine such as the spermidine is designed as the HA cross-linking agent, the entire HA gel is used as sustained release gel of the overall polyamine such as the spermidine, i.e., during the natural degradation of the gel in the body, the polyamine substance such as the spermidine may be released in a sustained prototypical manner. In the present disclosure, the three-site or four-site active star network gel may stabilize the release of a spermidine monomer under the enzymatic initiation of hyaluronidase, so as to continuously achieve the biological activity of the spermidine.

The present disclosure further provides a gel material obtained by the above preparation method.

In another aspect, the present disclosure further provides an application of the gel material or the gel material prepared by the preparation method in preparation of tissue filling and repair materials or drug carriers, for example, in products for drug preparation, medical cosmetology, and cosmetic purposes, for example, in terms of soft tissue filling and soft tissue repair, or cosmetic injections to eliminate facial skin wrinkles, etc.

Beneficial effects are as follows.

(1) According to the gel material provided in the present disclosure, the multi-site cross-linking between the endogenous polyamine and the HA is realized, so as to form the more compact network structure.
(2) After the HA gel obtained in the present disclosure is subjected to moist heat sterilization, the loss rate of the elastic modulus is low (which may be minimized to less than 10%), such that the rheological property of the gel before sterilization may be effectively maintained, thereby greatly improving the thermal stability of the HA gel.
(3) According to the preparation method of the present disclosure, the number of the cross-linking reaction sites may be controlled, such that the properties of the prepared cross-linked HA gel may be affected.
(4) In the present disclosure, the release rate of the endogenous polyamine such as the spermidine during the degradation of the HA gel may be controlled, so as to continuously achieving the physiologically active effects of the endogenous polyamine such as the spermidine.
(5) According to the present disclosure, the ease of use of the gel in fields such as soft tissue filling, soft tissue repair, medical cosmetology, etc. is improved, and is suitable for promotion and application.

## Brief Description of the Drawings

In order to more clearly illustrate the specific embodiments of the present disclosure or the technical solutions in the related art, the drawings used in the description of the specific embodiments or the related art will be briefly described below. It is apparent that the drawings in the following descriptions are some embodiments of the present disclosure. Other drawings can be obtained from those skilled in the art according to these drawings without any creative work.

Fig. 1 shows a $^1H$ NMR spectrum of spermidine cross-linked HA according to the present disclosure.

Fig. 2 shows a schematic diagram of cell proliferation of spermidine cross-linked HA according to the present disclosure.

Fig. 3 shows a curve of release of spermidine with cross-linked HA in spermidine cross-linked HA gel under a thermal degradation condition according to the present disclosure.

Fig. 4 shows a curve of release of spermidine with cross-linked HA in spermidine cross-linked HA gel under an enzymatic condition according to the present disclosure.

## Detailed Description of the Embodiments

The technical solutions of the present disclosure will be clearly and completely described below with reference to the embodiments. It is apparent that the described embodiments are merely part of the embodiments of the present disclosure, not all the embodiments. Based on the embodiments in the present disclosure, all other embodiments obtained by those of ordinary skill in the art without creative work shall fall within the protection scope of the present disclosure.

### Example 1: Preparation of HATU-catalyzed two-site cross-linked hydrogel of spermine and HA

3.0 g of sodium hyaluronate (a molecular weight being 900 KDa, and containing 7.4 mmol of a HA repeating structural unit) was weighed, then 98 mL of purified water was added, after complete dissolving, the current concentration of the HA was 30 mg/mL, 0.03 g (a mole number being 0.15 mmol) of spermine was added to the HA solution, and the current content of the spermine was 1% of the mass of the HA (2% of the mole number of the HA repeating structural unit). A pH value of the HA solution was regulated to about 6.20 by using a 6 mol/L hydrochloric acid solution, then uniformly stirred, 0.32 mmol of HATU was added, which was 213% of the mole number of the spermine, uniform stirring was continuously performed, and the mixture was sealed and placed in a 25°C incubator for reaction for 24 h. After the reaction ended, 40 mL of anhydrous ethanol was added, gel particles were smashed for 5 min by using an IKA T25 high shear disperser at a smashing rotary speed of 10,000 rpm. After smashing ended, 200 mL of the anhydrous ethanol was continuously added to make the gel completely dehydrated. Precipitation was separated, the precipitation was continuously washed for 5 times by using 200 mL of the anhydrous ethanol, then the precipitation was placed in a vacuum oven, and vacuum drying was performed for 24 h at 40°C and a vacuum degree of -0.09 MPa. After complete drying, 1.0 g of dry gel was taken, and 50 mL of a phosphate buffer solution with pH being 7.0 and concentration being 10 mg/mL was added in total, so as to prepare hydrogel with the concentration of the HA being 20 mg/mL; and after the gel was completely swelled, the gel was filled in a prefilled syringe, and moist heat sterilization was performed for 15 min at 121°C, so as to obtain a final product gel. The particle size D0.5 of the gel was 158 μm, and D0.9 was 196 μm.

### Example 2: Preparation of HATU-catalyzed three-site cross-linked hydrogel of spermidine and HA

3.0 g of the sodium hyaluronate (the molecular weight being 900 KDa, and containing 7.4 mmol of the HA repeating structural unit) was weighed, then 98 mL of the purified water was added, after complete dissolving, the current concentration of the HA was 30 mg/mL, 0.022 g (a mole number being 0.15 mmol) of spermidine was added to the HA solution, and the current content of the spermidine was 0.7% of the mass of the HA (2% of the mole number of the HA repeating structural unit). 0.48 mmol of the HATU was added, which was 320% of the mole number of the spermidine, uniform stirring was performed, the pH value of the HA solution was regulated to about 5.40 by using the 6 mol/L hydrochloric acid solution, then uniformly stirred, and the mixture was sealed and placed in the 50°C incubator for reaction for 14h. After the reaction ended, 40 mL of the anhydrous ethanol was added, the gel particles were smashed for 5 min by using the IKA T25 high shear disperser at the smashing rotary speed of 10,000 rpm. After smashing ended, 200 mL of the anhydrous ethanol was continuously added to make the gel completely dehydrated. The precipitation was separated, the precipitation was continuously washed for 5 times by using 200 mL of the anhydrous ethanol, then the precipitation was placed in the vacuum oven, and vacuum drying was performed for 24 h at 40°C and the vacuum degree of -0.09 MPa. After complete drying, 1.0 g of the dry gel was taken, and 50 mL of the phosphate buffer solution with pH being 7.0 and concentration being 10 mg/mL was added in total, so as to prepare the hydrogel with the concentration of the HA being 20 mg/mL; and after the gel was completely swelled, the gel was filled in the prefilled syringe, and moist heat sterilization was performed for 15 min at 121°C, so as to obtain the final product gel. The particle size D0.5 of the gel was 143 μm, and D0.9 was 201 μm.

### Example 3: Preparation of HATU-catalyzed four-site cross-linked hydrogel of spermine and HA

3.0 g of the sodium hyaluronate (the molecular weight being 900 KDa, and containing 7.4 mmol of the HA repeating structural unit) was weighed, then 98 mL of the purified water was added, after complete dissolving, the current concentration of the HA was 30 mg/mL, 0.03 g (the mole number being 0.15 mmol) of the spermine was added to the

HA solution, and the current content of the spermine was 1 % of the mass of the HA (2% of the mole number of the HA repeating structural unit). 0.65 mmol of the HATU was added, which was 433% of the mole number of the spermine, uniform stirring was performed, the pH value of the HA solution was regulated to about 5.30 by using the 6 mol/L hydrochloric acid solution, uniformly stirring was performed continuously, and the mixture was sealed and placed in the 60°C incubator for reaction for 14 h. After the reaction ended, 40 mL of acetone was added, the gel particles were smashed for 5 min by using the IKA T25 high shear disperser at the smashing rotary speed of 10,000 rpm. After smashing ended, 200 mL of the acetone was continuously added to make the gel completely dehydrated. The precipitation was separated, the precipitation was continuously washed for 5 times by using 200 mL of the acetone, then the precipitation was placed in the vacuum oven, and vacuum drying was performed for 24 h at 40°C and the vacuum degree of -0.09 MPa. After complete drying, 1.0 g of the dry gel was taken, and 50 mL of the phosphate buffer solution with pH being 7.0 and concentration being 10 mg/mL was added in total, so as to prepare the hydrogel with the concentration of the HA being 20 mg/mL; and after the gel was completely swelled, the gel was filled in the prefilled syringe, and moist heat sterilization was performed for 15 min at 121°C, so as to obtain the final product gel. The particle size D0.5 of the gel was 139 μm, and D0.9 was 186 μm.

**Example 4: Preparation of bromosuccinimide triphenylphosphine salt-catalyzed two-site cross-linked hydrogel of spermidine and HA**

0.2 mol of triphenylphosphine and 0.2 mol of N-hydroxysuccinimide were dissolved in 1000 mL of dichloromethane and stirred for 24h at 20-25°C; after the reaction completed, a rotary evaporator was used to remove the dichloromethane, so as to obtain a bromosuccinimide triphenylphosphine salt which was sealed for later use.

3.0 g of the sodium hyaluronate (the molecular weight being 900 KDa, and containing 7.4 mmol of the HA repeating structural unit) was weighed, then 98 mL of the purified water was added, after complete dissolving, the current concentration of the HA was 30 mg/mL, 0.022 g (a mole number being 0.15 mmol) of spermidine was added to the HA solution, and the current content of the spermidine was 0.7% of the mass of the HA (2% of the mole number of the HA repeating structural unit). 0.32 mmol of the bromosuccinimide triphenylphosphine salt obtained in the above step was added, which was 213% of the mole number of the spermidine, uniform stirring was performed, the pH value of the HA solution was regulated to about 6.00 by using the 6 mol/L hydrochloric acid solution, then uniformly stirred, and the mixture was sealed and placed in the 25°C incubator for reaction for 24h. After the reaction ended, 40 mL of acetone was added, the gel particles were smashed for 5 min by using the IKA T25 high shear disperser at the smashing rotary speed of 10,000 rpm. After smashing ended, 200 mL of the acetone was continuously added to make the gel completely dehydrated. The precipitation was separated, the precipitation was continuously washed for 5 times by using 200mL of the acetone, then the precipitation was placed in the vacuum oven, and vacuum drying was performed for 24h at 40°C and the vacuum degree of -0.09 MPa. After complete drying, 1.0 g of the dry gel was taken, and 50 mL of the phosphate buffer solution with pH being 7.0 and concentration being 10 mg/mL was added in total, so as to prepare the hydrogel with the concentration of the HA being 20 mg/mL; and after the gel was completely swelled, the gel was filled in the prefilled syringe, and moist heat sterilization was performed for 15 min at 121°C, so as to obtain the final product gel. The particle size D0.5 of the gel was 141 μm, and D0.9 was 191 μm.

**Example 5: Preparation of bromosuccinimide triphenylphosphine salt-catalyzed three-site cross-linked hydrogel of spermidine and HA**

3.0 g of the sodium hyaluronate (the molecular weight being 900 KDa, and containing 7.4 mmol of the HA repeating structural unit) was weighed, then 98 mL of the purified water was added, after complete dissolving, the current concentration of the HA was 30 mg/mL, 0.022 g (a mole number being 0.15 mmol) of spermidine was added to the HA solution, and the current content of the spermidine was 0.7% of the mass of the HA (2% of the mole number of the HA repeating structural unit). 0.48 mmol of the bromosuccinimide triphenylphosphine salt prepared according to the method in Example 4 was added, which was 320% of the mole number of the spermidine, uniform stirring was performed, the pH value of the HA solution was regulated to about 5.20 by using the 6 mol/L hydrochloric acid solution, then uniformly stirred, and the mixture was sealed and placed in the 40°C incubator for reaction for 24 h. After the reaction ended, 40 mL of acetone was added, the gel particles were smashed for 5 min by using the IKA T25 high shear disperser at the smashing rotary speed of 10,000 rpm. After smashing ended, 200 mL of the acetone was continuously added to make the gel completely dehydrated. The precipitation was separated, the precipitation was continuously washed for 5 times by using 200mL of the acetone, then the precipitation was placed in the vacuum oven, and vacuum drying was performed for 24 h at 40°C and the vacuum degree of -0.09 MPa. After complete drying, 1.0 g of the dry gel was taken, and 50 mL of the phosphate buffer solution with pH being 7.0 and concentration being 10 mg/mL was added in total, so as to prepare the hydrogel with the concentration of the HA being 20 mg/mL; and after the gel was completely swelled, the gel was filled in the prefilled syringe, and moist heat sterilization was performed for 15 min at 121°C, so as to obtain the final

product gel. The particle size D0.5 of the gel was 133 μm, and D0.9 was 178 μm.

**Example 6: Preparation of DMTMM-catalyzed three-site cross-linked hydrogel of spermidine and HA**

3.0 g of the sodium hyaluronate (the molecular weight being 900 KDa, and containing 7.4 mmol of the HA repeating structural unit) was weighed, then 98 mL of the purified water was added, after complete dissolving, the current concentration of the HA was 30 mg/mL, 0.022 g (a mole number being 0.15 mmol) of spermidine was added to the HA solution, and the current content of the spermidine was 0.7% of the mass of the HA (2% of the mole number of the HA repeating structural unit). 0.48 mmol of DMTMM was added, which was 320% of the mole number of the spermidine, uniform stirring was performed, the pH value of the HA solution was regulated to about 5.10 by using the 6 mol/L hydrochloric acid solution, then uniformly stirred, and the mixture was sealed and placed in the 40°C incubator for reaction for 24 h. After the reaction ended, 40 mL of the anhydrous ethanol was added, the gel particles were smashed for 5 min by using the IKA T25 high shear disperser at the smashing rotary speed of 10,000 rpm. After smashing ended, 200 mL of the anhydrous ethanol was continuously added to make the gel completely dehydrated. The precipitation was separated, the precipitation was continuously washed for 5 times by using 200 mL of the anhydrous ethanol, then the precipitation was placed in the vacuum oven, and vacuum drying was performed for 24 h at 40°C and the vacuum degree of -0.09 MPa. After complete drying, 1.0 g of the dry gel was taken, and 50 mL of the phosphate buffer solution with pH being 7.0 and concentration being 10 mg/mL was added in total, so as to prepare the hydrogel with the concentration of the HA being 20 mg/mL; and after the gel was completely swelled, the gel was filled in the prefilled syringe, and moist heat sterilization was performed for 15 min at 121°C, so as to obtain the final product gel. The particle size D0.5 of the gel was 144 μm, and D0.9 was 206 μm.

**Example 7: Preparation of EDC-catalyzed four-site cross-linked hydrogel of spermine and HA**

3.0 g of the sodium hyaluronate (the molecular weight being 900 KDa, and containing 7.4 mmol of the HA repeating structural unit) was weighed, then 98 mL of the purified water was added, after complete dissolving, the current concentration of the HA was 30 mg/mL, 0.03 g (the mole number being 0.15 mmol) of the spermine was added to the HA solution, and the current content of the spermine was 1% of the mass of the HA (2% of the mole number of the HA repeating structural unit). 0.65 mmol of EDC was added, which was 433% of the mole number of the spermine, NHS of which mass is 20% of the mass of the EDC was added, uniform stirring was performed, the pH value of the HA solution was regulated to about 5.45 by using the 6 mol/L hydrochloric acid solution, uniformly stirring was performed continuously, and the mixture was sealed and placed in the 60°C incubator for reaction for 14 h. After the reaction ended, 40 mL of the anhydrous ethanol was added, the gel particles were smashed for 5 min by using the IKAT25 high shear disperser at the smashing rotary speed of 10,000 rpm. After smashing ended, 200 mL of the anhydrous ethanol was continuously added to make the gel completely dehydrated. The precipitation was separated, the precipitation was continuously washed for 5 times by using 200 mL of the anhydrous ethanol, then the precipitation was placed in the vacuum oven, and vacuum drying was performed for 24 h at 40°C and the vacuum degree of -0.09 MPa. After complete drying, 1.0 g of the dry gel was taken, and 50 mL of the phosphate buffer solution with pH being 7.0 and concentration being 10 mg/mL was added in total, so as to prepare the hydrogel with the concentration of the HA being 20 mg/mL; and after the gel was completely swelled, the gel was filled in the prefilled syringe, and moist heat sterilization was performed for 15 min at 121°C, so as to obtain the final product gel. The particle size D0.5 of the gel was 125 μm, and D0.9 was 193 μm.

**Example 8: Preparation of HATU-catalyzed multi-site cross-linked hydrogel of HA with participation of spermidine and spermine in combined use manner**

3.0 g of the sodium hyaluronate (the molecular weight being 900 KDa, and containing 7.4 mmol of the HA repeating structural unit) was weighed, then 98 mL of the purified water was added, after complete dissolving, the current concentration of the HA was 30 mg/mL, 0.022 g (the mole number being 0.15 mmol) of the spermidine and 0.03 g (the mole number being 0.15 mmol) of the spermine were respectively added to the HA solution, and the current content of the spermidine or spermine was 2% of the mole number of the HA repeating structural unit. 0.48 mmol of the HATU was added, which was 320% of the mole number of the spermidine, then 0.65 mmol of the HATU was added, which was 433% of the mole number of the spermine, uniform stirring was performed, the pH value of the HA solution was regulated to about 5.00 by using the 6 mol/L hydrochloric acid solution, then uniformly stirred, and the mixture was sealed and placed in the 50 °C incubator for reaction for 14 h. After the reaction ended, 40 mL of the anhydrous ethanol was added, the gel particles were smashed for 5 min by using the IKA T25 high shear disperser at the smashing rotary speed of 10,000 rpm. After smashing ended, 200 mL of the anhydrous ethanol was continuously added to make the gel completely dehydrated. The precipitation was separated, the precipitation was continuously washed for 5 times by using 200 mL of the anhydrous ethanol, then the precipitation was placed in the vacuum oven, and vacuum drying was performed for

24 h at 40 °C and the vacuum degree of -0.09 MPa. After complete drying, 1.0 g of the dry gel was taken, and 50 mL of the phosphate buffer solution with pH being 7.0 and concentration being 10 mg/mL was added in total, so as to prepare the hydrogel with the concentration of the HA being 20 mg/mL; and after the gel was completely swelled, the gel was filled in the prefilled syringe, and moist heat sterilization was performed for 15 min at 121 °C, so as to obtain the final product gel. The particle size D0.5 of the gel was 134 μm, and D0.9 was 236 μm.

**Example 9: EDC-catalyzed three-site cross-linking of spermidine and HA**

6 g of the sodium hyaluronate (the molecular weight being 120 KDa) was weighed, then 35 mL of the purified water was added, after complete dissolving, the current concentration of the HA was 150 mg/mL, 2.4 g (a mole number being 16.6 mmol) of spermidine was added to the HA solution, and the current content of the spermidine was 40% of the mass of the HA. Under the premise of three-site cross-linking, the pH value of the HA solution was regulated to about 5.2 by using the 6 mol/L hydrochloric acid solution, then uniformly stirred, 66.4 mmol of the EDC was added, which was 400% of the mole number of the spermidine, 2.4 g (20% of the mass of the EDC) of Sulfo-NHS was added at the same time, uniformly stirring was performed continuously, and the mixture was sealed and placed in a 60 °C drying box for reaction for 24 h. After the reaction ended, 40 mL of the anhydrous ethanol was added, the gel particles were smashed for 5 min by using the IKA T25 high shear disperser at the smashing rotary speed of 10,000 rpm. After smashing ended, 200 mL of the anhydrous ethanol was continuously added to make the gel completely dehydrated. The precipitation was separated, the precipitation was continuously washed for 5 times by using 200mL of the anhydrous ethanol, then the precipitation was placed in the vacuum oven, and vacuum drying was performed for 24 h at 40 °C and the vacuum degree of -0.09 MPa. After complete drying, 1.0 g of the dry gel was taken, and 20 mL of the phosphate buffer solution with pH being 7.0 and concentration being 10 mg/mL was added in total; and after the gel was completely swelled, the gel was filled in the prefilled syringe, and moist heat sterilization was performed for 15 min at 121°C, so as to obtain the final product gel. The particle size D0.5 of the gel was 145 μm, and D0.9 was 213 μm.

**Example 10: Preparation of HATU-catalyzed four-site cross-linked hydrogel of spermine and HA**

0.5 g of the sodium hyaluronate (the molecular weight being 2800 KDa) was weighed, then 38.5 mL of the purified water was added, after complete dissolving, the current concentration of the HA was 13.3 mg/mL, 1.5mg (the mole number being 7.4 μmol) of the spermine was added to the HA solution, and the current content of the spermine was 0.3% of the mass of the HA. Under the premise of four-site cross-linking, the pH value of the HA solution was regulated to about 5.2 by using the 6 mol/L hydrochloric acid solution, then uniformly stirred, 29.6 μmol of the HATU was added, which was 400% of the mole number of the spermine, uniformly stirring was performed continuously, and the mixture was sealed and placed in the 30 °C drying box for reaction for 24 h. After the reaction ended, 40 mL of acetone was added, the gel particles were smashed for 5 min by using the IKAT25 high shear disperser at the smashing rotary speed of 10,000 rpm. After smashing ended, 200 mL of the acetone was continuously added to make the gel completely dehydrated. The precipitation was separated, the precipitation was continuously washed for 5 times by using 200mL of the acetone, then the precipitation was placed in the vacuum oven, and vacuum drying was performed for 24 h at 40 °C and the vacuum degree of -0.09 MPa. After complete drying, 0.4 g of the dry gel was taken, and 400 mL of the phosphate buffer solution with pH being 7.0 and concentration being 10 mg/mL was added in total; and after the gel was completely swelled, the gel was filled in the prefilled syringe, and moist heat sterilization was performed for 15 min at 121 °C, so as to obtain the final product gel. The particle size D0.5 of the gel was 156 μm, and D0.9 was 231 μm.

**Example 11: Preparation of HATU-catalyzed three-site cross-linked hydrogel of spermidine and HA**

0.5 g of the sodium hyaluronate (the molecular weight being 2800 KDa) was weighed, then 38.5 mL of the purified water was added, after complete dissolving, the current concentration of the HA was 13.3 mg/mL, 2.5 mg (the mole number being 17.2 μmol) of the spermidine was added to the HA solution, and the current content of the spermidine was 0.5% of the mass of the HA. Under the premise of three-site cross-linking, the pH value of the HA solution was regulated to about 5.2 by using the 6 mol/L hydrochloric acid solution, then uniformly stirred, 51.6 μmol of the HATU was added, which was 300% of the mole number of the spermidine, uniformly stirring was performed continuously, and the mixture was sealed and placed in the 30 °C drying box for reaction for 24 h. After the reaction ended, 40 mL of acetone was added, the gel particles were smashed for 5 min by using the IKA T25 high shear disperser at the smashing rotary speed of 10,000 rpm. After smashing ended, 200 mL of the acetone was continuously added to make the gel completely dehydrated. The precipitation was separated, the precipitation was continuously washed for 5 times by using 200 mL of the acetone, then the precipitation was placed in the vacuum oven, and vacuum drying was performed for 24 h at 40 °C and the vacuum degree of - 0.09 MPa. After complete drying, 0.4 g of the dry gel was taken, and 200 mL of the phosphate buffer solution with pH being 7.0 and concentration being 10 mg/mL was added in total; and after the

gel was completely swelled, the gel was filled in the prefilled syringe, and moist heat sterilization was performed for 15 min at 121 °C, so as to obtain the final product gel. The particle size D0.5 of the gel was 125 μm, and D0.9 was 201 μm.

**Example 12: EDC-catalyzed four-site cross-linking of spermine and HA**

6 g of the sodium hyaluronate (the molecular weight being 120 KDa) was weighed, then 35 mL of the purified water was added, after complete dissolving, the current concentration of the HA was 150 mg/mL, 2.1 g (the mole number being 10.4 mmol) of spermine was added to the HA solution, and the current content of the spermine was 35% of the mass of the HA. Under the premise of four-site cross-linking, the pH value of the HA solution was regulated to about 5.2 by using the 6 mol/L hydrochloric acid solution, then uniformly stirred, 57.2 mmol of the EDC was added, which was 550% of the mole number of the spermine, 2.0 g (20% of the mass of the EDC) of HOBt was added at the same time, uniformly stirring was performed continuously, and the mixture was sealed and placed in the 60 °C drying box for reaction for 24 h. After the reaction ended, 40 mL of the anhydrous ethanol was added, the gel particles were smashed for 5 min by using the IKA T25 high shear disperser at the smashing rotary speed of 10,000 rpm. After smashing ended, 200 mL of the anhydrous ethanol was continuously added to make the gel completely dehydrated. The precipitation was separated, the precipitation was continuously washed for 5 times by using 200 mL of the anhydrous ethanol, then the precipitation was placed in the vacuum oven, and vacuum drying was performed for 24 h at 40 °C and the vacuum degree of -0.09 MPa. After complete drying, 1.0 g of the dry gel was taken, and 20mL of the phosphate buffer solution with pH being 7.0 and concentration being 10 mg/mL was added in total; and after the gel was completely swelled, the gel was filled in the prefilled syringe, and moist heat sterilization was performed for 15 min at 121 °C, so as to obtain the final product gel. The particle size D0.5 of the gel was 148 μm, and D0.9 was 197 μm.

**Example 13: Preparation of HATU-catalyzed two-site cross-linked hydrogel of spermidine and HA under acidic condition**

3.0 g of the sodium hyaluronate (the molecular weight being 900 KDa, and containing 7.4 mmol of the HA repeating structural unit) was weighed, then 98mL of the purified water was added, after complete dissolving, the current concentration of the HA was 30 mg/mL, 0.022g (a mole number being 0.15 mmol) of spermidine was added to the HA solution, and the current content of the spermidine was 0.7% of the mass of the HA (2% of the mole number of the HA repeating structural unit). 0.48 mmol of the HATU was added, which was 320% of the mole number of the spermidine, uniform stirring was performed, the pH value of the HA solution was regulated to about 4.60 by using the 6mol/L hydrochloric acid solution, then uniformly stirred, and the mixture was sealed and placed in the 50°C incubator for reaction for 14 h. After the reaction ended, 40 mL of the anhydrous ethanol was added, the gel particles were smashed for 5 min by using the IKAT25 high shear disperser at the smashing rotary speed of 10,000 rpm. After smashing ended, 200 mL of the anhydrous ethanol was continuously added to make the gel completely dehydrated. The precipitation was separated, the precipitation was continuously washed for 5 times by using 200mL of the anhydrous ethanol, then the precipitation was placed in the vacuum oven, and vacuum drying was performed for 24 h at 40°C and the vacuum degree of -0.09MPa. After complete drying, 1.0 g of the dry gel was taken, and 50 mL of the phosphate buffer solution with pH being 7.0 and concentration being 10 mg/mL was added in total, so as to prepare the hydrogel with the concentration of the HA being 20 mg/mL; and after the gel was completely swelled, the gel was filled in the prefilled syringe, and moist heat sterilization was performed for 15 min at 121°C, so as to obtain the final product gel. The particle size D0.5 of the gel was 138 μm, and D0.9 was 210 μm.

**Comparative example 1: Preparation of HATU-catalyzed spermidine and HA cross-linked hydrogel under strong acid condition**

3.0 g of the sodium hyaluronate (the molecular weight being 900 KDa, and containing 7.4 mmol of the HA repeating structural unit) was weighed, then 98 mL of the purified water was added, after complete dissolving, the current concentration of the HA was 30 mg/mL, 0.022g (a mole number being 0.15 mmol) of spermidine was added to the HA solution, and the current content of the spermidine was 0.7% of the mass of the HA (2% of the mole number of the HA repeating structural unit). 0.48 mmol of the HATU was added, which was 320% of the mole number of the spermidine, uniform stirring was performed, the pH value of the HA solution was regulated to about 3.20 by using the 6mol/L hydrochloric acid solution, then uniformly stirred, and the mixture was sealed and placed in the 50°C incubator for reaction for 14 h. After the reaction ended, 40 mL of the anhydrous ethanol was added, the gel particles were smashed for 5 min by using the IKA T25 high shear disperser at the smashing rotary speed of 10,000 rpm. After smashing ended, 200 mL of the anhydrous ethanol was continuously added to make the gel completely dehydrated. The precipitation was separated, the precipitation was continuously washed for 5 times by using 200 mL of the anhydrous ethanol, then the precipitation was placed in the vacuum oven, and vacuum drying was performed for 24 h at 40 °C and the vacuum degree

of -0.09MPa. After complete drying, 1.0 g of the dry gel was taken, and 50 mL of the phosphate buffer solution with pH being 7.0 and concentration being 10 mg/mL was added in total, so as to prepare the hydrogel with the concentration of the HA being 20 mg/mL; and after the gel was completely swelled, the gel was filled in the prefilled syringe, and moist heat sterilization was performed for 15 min at 121°C, so as to obtain the final product gel. The particle size D0.5 of the gel was 143 μm, and D0.9 was 201 μm.

**Comparative example 2: EDC-catalyzed cross-linking reaction between spermine and HA**

In this comparative example, gel preparation was performed according to the method disclosed in US9907739B2.

3.0 g of the sodium hyaluronate (the molecular weight being 900 KDa, and containing 7.4 mmol of the HA repeating structural unit) was weighed, then 98 mL of the purified water was added, after complete dissolving, the current concentration of the HA was 30 mg/mL, 0.03 g (the mole number being 0.15 mmol) of the spermine was added to the HA solution, and the current content of the spermine was 1% of the mass of the HA (2% of the mole number of the HA repeating structural unit). The pH value of the HA solution was regulated to about 6.2 by using the 6mol/L hydrochloric acid solution and uniformly stirred, 7.4 mmol of the EDC was added, which was 100% of the mole number of the HA structural unit and 49 times of the mole number of the spermine, the HOBt of which mass is 20% of the mass of the EDC was added at the same time, uniform stirring was continuously performed, and the mixture was sealed and placed in the 25°C incubator for reaction for 24 h. After the reaction ended, 40 mL of the anhydrous ethanol was added, the gel particles were smashed for 5 min by using the IKAT25 high shear disperser at the smashing rotary speed of 10,000 rpm. After smashing ended, 200 mL of the anhydrous ethanol was continuously added to make the gel completely dehydrated. The precipitation was separated, the precipitation was continuously washed for 5 times by using 200 mL of the anhydrous ethanol, then the precipitation was placed in the vacuum oven, and vacuum drying was performed for 24 h at 40 °C and the vacuum degree of -0.09 MPa. After complete drying, 1.0 g of the dry gel was taken, and 50 mL of the phosphate buffer solution with pH being 7.0 and concentration being 10 mg/mL was added in total, so as to prepare the hydrogel with the concentration of the HA being 20 mg/mL; and after the gel was completely swelled, the gel was filled in the prefilled syringe, and moist heat sterilization was performed for 15 min at 121°C, so as to obtain the final product gel. The particle size D0.5 of the gel was 155 μm, and D0.9 was 221 μm.

**Example 14: Detection of cross-linking efficiency of spermine or spermidine**

2.0 mL of the HA gel obtained in Examples 1-8 and Comparative examples 1-2 was taken out, respectively, 50 mL of the anhydrous ethanol was added to each portion of the gel, and stirring was performed for 30 min until the gel changed to white precipitation. The precipitation was separated, and vacuum drying was performed for 24 h at 40°C and the vacuum degree of -0.09 MPa, so as to remove the ethanol. The gel was taken out and added to 10 mL of a 0.5 mol/L sulfuric acid solution, respectively, was treated for 2 h at 90°C, and then was neutralized to pH=7 by using a 6 mol/L sodium hydroxide solution. The solution was placed in the vacuum oven, and vacuum drying was performed for 24 h at 60 °C and the vacuum degree of -0.09MPa, so as to remove excess moisture. The mixture was dissolved in deuterated water with the concentration being 10 mg/mL, and $^1$H NMR spectrum scanning was performed by using a 400 M nuclear magnetic resonance spectrometer.

Through integration of key characteristic peaks in a $^1$H NMR spectrum, an actual molar ratio of the spermidine to the HA was obtained through an integral peak area, and the cross-linking efficiency was obtained by comparing the actual molar ratio with a theoretical molar ratio (which was calculated according to actual feeding). Characteristic H atoms selected in the spermidine were α-H of the amino group or imino group, and there were 8 H atoms in total; the characteristic H atoms selected in the spermine were α-H of the amino group or imino group, and there were 12 H atoms in total; the H atoms of the spermine or spermidine had the same integration range, which was 2.5-2.85 ppm; the characteristic H atoms selected in the HA were α-H of carbonyl of acetylamino, there were 3 H atoms in total, and the integration range was 1.85-2.05 ppm; and during integration, the integral peak area of the carbonyl α-H in the HA was set to 3, such that the integral peak area of the α-H of the amino group or imino group in the spermidine or spermine might be obtained. A calculation formula was shown as follows.

Actual cross-linking molar ratio of spermidine = integral peak area of α-H of amino group and imino group in spermidine/8

Actual cross-linking molar ratio of spermine = integral peak area of α-H of amino group and imino group in spermine/12

Cross-linking efficiency = (actual cross-linking molar ratio/theoretical cross-linking molar ratio) $\times$ 100%

The cross-linking efficiency of Examples 1-8 was shown in Table 1, and a nuclear magnetic spectrum was shown in Fig. 1.

Table 1 Actual cross-linking efficiency

| Sample | Theoretical cross-linking molar ratio | Actual cross-linking molar ratio | Cross-linking efficiency |
|---|---|---|---|
| Example 1 | 2.0% | 1.7% | 85% |
| Example 2 | 2.0% | 1.9% | 95% |
| Example 3 | 2.0% | 1.9% | 95% |
| Example 4 | 2.0% | 1.5% | 75% |
| Example 5 | 2.0% | 1.7% | 85% |
| Example 6 | 2.0% | 1.6% | 80% |
| Example 7 | 2.0% | 1.5% | 75% |
| Example 8 | 4.0% | 3.9% | 97% |
| Comparative example 1 | 2.0% | 0.9% | 45% |
| Comparative example 2 | 2.0% | 1.0% | 50% |

By analyzing data in Table 1, it was found that, during two-site cross-linking, the cross-linking efficiency was lower than that of multi-site cross-linking. When Examples 1 and 4 in which two-site cross-linking was performed were compared with Examples 2, 3, and 5, it was found that the efficiency of the two-site cross-linking was generally lower than that of the multi-site cross-linking, and this might be due to a combined effect of the use amount of the activating agent, the pH value, and the temperature, which led to low cross-linking efficiency during the two-site cross-linking. Likewise, during the multi-site cross-linking, the sequence of the efficiency of each activating agent to perform activation was: HATU > triphenylphosphine salt > DMTMM > EDC + additive. The activation efficiency of the HATU might be up to nearly 95%, and the activation efficiency of the EDC+NHS was only 75%. Under the same conditions, the cross-linking efficiency of the spermine and the spermidine was close to each other, and due to high content of the imino group in the spermine, the efficiency of the spermine was slightly lower; and due to large spatial steric hindrance of the imino group, the reaction activity was lower than that of the primary amino group. In addition, by analyzing the comparative examples, beyond the multi-site pH value range, the reaction activity of the amino and imino groups was suppressed, such that the cross-linking efficiency was relatively low; and at a test temperature lower than the multi-site cross-linking, even if the reaction was performed according to the conditions of the multi-site cross-linking, the finally obtained cross-linking efficiency was close to that of the two-site cross-linking, and the reaction efficiency was lower.

**Example 15: Impact of spermidine cross-linked HA gel on cell proliferation**

L-929 cells were planted in a cell culture medium, 1% of a penicillin-streptomycin solution and 10% of a fetal bovine serum solution were added. The L-929 cells were incubated for 3 days at a constant temperature of 37°C in a moist cell incubator containing 5% carbon dioxide. Then the spermidine three-site cross-linked HA gel obtained in Example 2 was transferred in a 96-well plate, cured and sterilized by using an ultraviolet lamp. Then, the L-929 cell culture fluid was added to wells in which the spermidine cross-linked HA gel was placed, and 1 mL of a trypsin solution containing 0.1% EDTA was added; and the number of the L-929 cells in each well was $1\times10^5$. The cells were continuously placed in the cell incubator to promote cell growth.

A cell proliferation rate of the spermidine cross-linked HA gel was determined by an MTT method. After 24, 48, and 72 hours of cultivation respectively, 100 $\mu$L of an MTT aqueous solution (with the concentration being 5 $mg\cdot mL^{-1}$) was added to each well, and placed in the incubator for continuous cultivation for 4 hours. Then the MTT solution was removed, 150 $\mu$L of dimethyl sulfoxide was added to dissolve formazan crystals, and the absorbance of the solution was detected at a wave length of 490nm by using a microplate reader, so as to determine a cell proliferation degree of the gel to the L-929. A cell survival rate was calculated according to the following formula.

$$\text{Cell survival rate (\%)} = (As/Ac) \times 100\%.$$

As was the absorbance of a sample solution at 570 nm, and Ac was the absorbance of a blank control at 570 nm.

Cell proliferation results were shown in Fig. 2. From the figure, it might be seen that, the cell survival rates of the spermidine cross-linked HA gel at 24, 48, and 72 hours were respectively 112%, 123%, and 138%. From the cell survival rates of the two kinds of gel, it might be seen that, the spermidine cross-linked HA gel had no cytotoxicity, and the increase in the cell survival rate over time indicated that the hydrogel played a role in promoting cell growth to a certain extent.

**Example 16: Detection of rheological property of spermine or spermidine cross-linked HA gel**

The HA gel obtained in Examples 1-13 and Comparative example 2 was divided into two types before and after sterilization, 2.0 mL of each the two types was taken, the elastic modulus (G') of the gel was detected by using a TA DHR-2 flat plate rheometer, and the loss rate of the elastic modulus was calculated. The G' loss rate was calculated by the following formula.

$$\text{G' loss rate} = (\text{G' before sterilization} - \text{G' after sterilization})/\text{G' before sterilization}$$

Parameters of the rheometer included: operation gap: 1000 mm; loading gap: 45000 m; operating temperature: 37 °C; deformation quantity: 1%; frequency: 0.9 Hz; and operating time: 60 s. Gel rheology data was shown in Table 2.

Table 2 Rheology data of gel

| Sample | G' before sterilization (Pa) | G' after sterilization (Pa) | G' loss rate |
|---|---|---|---|
| Example 1 | 466 | 411 | 11.8% |
| Example 2 | 533 | 494 | 7.3% |
| Example 3 | 632 | 588 | 7.0% |
| Example 4 | 448 | 388 | 13.4% |
| Example 5 | 505 | 459 | 9.1% |
| Example 6 | 485 | 433 | 10.7% |
| Example 7 | 581 | 512 | 11.9% |
| Example 8 | 1005 | 958 | 4.7% |
| Example 9 | 843 | 725 | 14.0% |
| Example 10 | 0.82 | 0.70 | 14.6% |
| Example 11 | 1.54 | 1.31 | 14.9% |
| Example 12 | 985 | 878 | 10.9% |
| Example 13 | 455 | 403 | 11.4% |
| Comparative example 2 | 405 | 311 | 23.2% |

The G' loss rate might be considered as a characteristic index of the thermal stability of the gel; and if the G' loss rate was lower, the thermal stability of the gel was higher. From the data in Table 2, it might be seen that, using Examples 1-7 as examples, the G' loss rate was related to the cross-linking efficiency in Example 14 to a certain extent. With the similar cross-linking degree and cross-linking efficiency (Examples 2 and 3), the thermal stability of the spermidine cross-linked HA was similar to that of the spermine cross-linked HA, but the spermine had higher elastic modulus and higher thermal stability, this might because the cross-linked network structure formed by the 4-site cross-linking of the spermine was more compact, and the same theory might be applied to the three-site cross-linked spermidine gel and two-site cross-linked spermidine gel, i.e., the thermal stability of the two-site cross-linked gel was worse than that of the three-site cross-linked gel. By comparing four activating agents, the sequence of the thermal stability of the gel was: HATU > triphenylphosphine salt > DMTMM > EDC + additive, which was the same as Example 14. In addition, the

concentration of the HA in the cross-linked HA hydrogel had large impact on the thermal stability of the gel. Examples 10 and 11 used the same cross-linking sites as Examples 2 and 3. However, since the concentrations of the HA in the cross-linked HA hydrogel in Examples 10 and 11 respectively were 1 mg/mL and 2 mg/mL, and the corresponding concentrations in Examples 2 and 3 were 20 mg/mL, results of a large difference in G' and an increase in the G' decreased value were caused. In addition, the addition amount of the activating agent also had large impact on the properties of the hydrogel. Under the same conditions in Examples 1 and 13, the addition amount of the HATU activating agent in Example 13 was reduced by 28.6% (i.e., 164.8 μmol) compared to Example 1, resulting in a significant decrease in G' in Example 13 as compared to Example 1 and a significant increase in the proportion of decrease in G' as compared to Example 1.

**Example 17: Release detection of degradation of spermidine with HA in gel under thermal degradation condition**

The finished gel in Example 2 was taken, with 2mL each sample; the gel was sealed in a vial, and then placed in the drying box at 125 °C to accelerate hydrolysis; 3 parallel samples were taken every 15 min; after the samples were cooled to about 257 °C, the pHs were respectively regulated to 12 by using sodium hydroxide, and 20 mL of chromatographic grade acetone was added respectively; after filtration was performed by using a 0.2 μm filter membrane, the acetone filtrate was sampled to an Agilent 7890B gas chromatograph, and a SGE H-2 capillary column (30 m×0.53 mm×1.0 μm) was used; and chromatographic conditions included: inlet temperature: 220°C; pressure: 40; separation flow rate: 30; split ratio: 10:1; separation time: 0.8; column temperature: 100°C, maintaining the temperature for 0.5 min, rising the temperature to 180 °C at 20 °C/min, and maintaining the temperature for 1 min; and detector (FID): temperature: 250 °C, air flow rate: 350 mL/min, $H_2$ flow rate: 35 mL/min, and make-up $N_2$ flow rate: 30 mL/min.

Total degradation time was 90 min, 6 detection points were set in total, and detection results were shown in Fig. 3.

This experiment was intended to simulate a process of releasing a spermidine monomer during the thermal degradation of the HA due to the hydrolysis of cross-linking sites. However, since, in a normal physiological environment, the degradation process of the cross-linked HA took a long time, ranging from a few months to more than 1 year, this experiment used a release detection experiment for the spermidine during the hydrolysis acceleration process of the spermidine cross-linked HA gel at a high temperature higher than 100°C, such that the HA degradation process and the spermidine release process might be observed within a short time. As shown in Fig. 3, the spermidine slowly released with the degradation of the HA; initially, the release rate was slow, and later, with the disintegration of the cross-linked HA network, the release rate of the spermidine accelerated significantly, and the cumulative release also increased significantly, indicating that the spermidine in the gel showed a process of slowly releasing first and then quickly releasing. This experiment might also prove that the cross-linking technology in the present disclosure was an active reaction technology, the spermidine monomer might be released in a sustained prototypical manner during the continuous degradation of the cross-linked HA, and it might thus indicate that the gel might continuously release the spermidine in the body and achieved the specific physiologically active effect of the spermidine.

**Example 18: Release detection of degradation of spermidine with HA in gel under enzymatic condition**

The finished gel in Example 2 was taken, with 2 mL each sample; the gel was sealed in the vial, and 1000 u of hyaluronidase was added to each sample to accelerate hydrolysis; 3 parallel samples were taken every 30 min; after the samples were cooled to about 25 °C, 0.5 mL of supernatant was taken, the pHs were respectively regulated to 12 by using sodium hydroxide, and 20 mL of the chromatographic grade acetone was added respectively; after filtration was performed by using the 0.2 μm filter membrane, the acetone filtrate was sampled to the Agilent 7890B gas chromatograph, and the SGE H-2 capillary column (30 m×0.53 mm×1.0 μm) was used; and the chromatographic conditions included: inlet temperature: 220 °C; pressure: 40; separation flow rate: 30; split ratio: 10:1; separation time: 0.8; column temperature: 100 °C, maintaining the temperature for 0.5 min, rising the temperature to 180 °C at 20 °C/min, and maintaining the temperature for 1 min; and detector (FID): temperature: 250 °C, air flow rate: 350 mL/min, $H_2$ flow rate: 35 mL/min, and make-up $N_2$ flow rate: 30 mL/min.

Total degradation time was 180 min, 6 detection points were set in total, and detection results were shown in Fig. 4.

This experiment was intended to simulate the process of releasing the spermidine monomer during the enzymatic degradation of the HA due to the degradation of the gel. As shown in Fig. 4, the spermidine slowly released with the degradation of the HA; during the overall degradation of the HA gel, the release rate of the spermine was almost constant, indicating that, during the enzymatic degradation of the HA obtained using spermidine cross-linking, the spermidine might be uniformly and stably released. This experiment might also prove that the cross-linking technology in the present disclosure was an active reaction technology, the spermidine monomer might be released in a sustained prototypical manner during the continuous degradation of the cross-linked HA, and it might thus indicate that the gel might continuously release the spermidine in the body and achieved the specific physiologically active effect of the

spermidine.

**Example 19: Detection of remaining active sites in two-site and multi-site cross-linked gel**

Approximately 2 g of ninhydrin hydrate was taken, 100 mL of the purified water was added to dissolve the ninhydrin hydrate, and well mixed; and the mixture was preserved under dark at 2-8°C for later use. 54.6 g of sodium acetate was taken, 20 mL of a 1 mol/L acetic acid solution was added to dissolve the sodium acetate, and water was added to dilute the mixture to 500 mL for later use. A spermidine standard and a spermine standard were respectively taken, placed in a 10 mL measuring bottle, diluted to scale with the purified water, and well shaken, so as to prepare a 500 μg/mL spermidine standard use solution and a 500 μg/mL spermine standard use solution; and when being used, the solutions were quantitatively diluted to 10, 50, 100, 200 μg/mL, and an original 500 μg/mL standard use solution was used as a standard curve.

The hydrogel in Examples 1-8 and Comparative examples 1 and 2 before moist heat sterilization and the spermine and spermidine series standard solutions were taken, 1 mL of each of the above was taken, 2.0 mL of an acetic acid-sodium acetate buffer solution and 2.0 mL of the ninhydrin hydrate solution were added in sequence, plugs were added, after well mixing, the mixture was placed in a 70 °C water bath for heating for 30 min, taken out and immediately cooled to room temperature, diluted to 25 mL with the purified water, and well mixed; the mixed solution was taken, the absorbance of an amino group derivative at the wave length of 565nm was determined, and the absorbance of an imino group derivative at the wave length of 400nm was detected at the same time; and blank correction was performed with the purified water by using the same method.

The proportion (%) of amino or imino residues was calculated according to the following formula.

$$\text{Residue proportion} = (A_0\text{-}A_i)/A_0 \times 100\%.$$

In the formula, $A_0$ was an absorbance value obtained by measuring a control solution, and $A_i$ was an absorbance value obtained by measuring a sample.

One spermidine molecule contained one imino group and two amino groups, and one spermine molecule contained two imino groups and two amino groups.

Actual detection results were shown in Table 3.

Table 3 Proportion of amino and imino residues in gel

| Sample | Amino residues | Imino residues |
|---|---|---|
| Example 1 | 10.34% | 86.61% |
| Example 2 | 7.31% | 8.02% |
| Example 3 | 8.53% | 9.45% |
| Example 4 | 18.13% | 89.45% |
| Example 5 | 10.16% | 12.31% |
| Example 6 | 13.35% | 13.43% |
| Example 7 | 17.41% | 14.31% |
| Example 8 | 6.32% | 7.88% |
| Comparative example 1 | 39.25% | 97.13% |
| Comparative example 2 | 40.35% | 98.31% |

From the results of Table 3, it might be learned that, Examples 1 and 4 were the two-site cross-linking, with a main reaction group being the amino group, the reaction efficiency was up to 89.66% (100%-amino residues, same below), the lowest amino reaction efficiency also exceeded 80%, and imino reaction efficiency did not exceed 15%, such that, under this condition, it was mainly the two-site cross-linking reaction involving the amino group. In Examples 2, 3, and 8, the reaction efficiency of the amino and imino groups of the spermidine or spermine reached more than 90%. In Examples 5-7, the reaction efficiency of the imino group also exceeded 85%. It indicated that, under this condition, the three-site cross-linking occurred in most of the spermidine, and the four-site cross-linking occurred in the spermine. Therefore, the authenticity of three-site and four-site cross-linking in the present disclosure was further verified. Through

the detection of the comparative examples, it was found that, beyond the conditions limited in the present disclosure, the imino reaction efficiency in the spermidine or spermine reduced to no more than 10%, and the reaction efficiency of the amino group also reduced. Although the total addition amount of the activating agent in Comparative example 2 was 49 times that of the cross-linking agent, the imino group in the spermine failed to participate in the reaction due to inappropriate reaction conditions such as pH and reaction temperatures, and the reaction efficiency of the amino group did not exceed 60%.

Finally, it should be noted that, the above embodiments are merely for describing and not intended to limit the technical solutions of the present disclosure. Although the present disclosure has been described in detail with reference to the above embodiments, those of ordinary skill in the art should understand that, they can still make modifications to the technical solutions recited in the above embodiments or make equivalent replacements to a part or all of the technical features thereof; and the modifications or replacements do not cause essence of the corresponding technical solutions to depart from the scope of the technical solutions of the embodiments of the present disclosure.

## Claims

1. A gel material, wherein the gel material is obtained by cross-linking endogenous polyamine with HA, and the endogenous polyamine comprises spermine and/or spermidine; the cross-linking between the endogenous polyamine and the HA comprises two-site cross-linking, three-site cross-linking, or four-site cross-linking;

    the proportion of amino residues in the gel obtained through the two-site cross-linking is lower than 20%; and
    the proportions of amino residues and imino residues in the gel obtained through the three-site cross-linking or four-site cross-linking are all lower than 20%.

2. The gel material according to claim 1, wherein the proportion of the amino residues in the gel obtained through the cross-linking in the two-site cross-linking, three-site cross-linking, or four-site cross-linking is lower than 15%; and further, the proportion of the amino residues in the gel obtained through the cross-linking in the two-site cross-linking, three-site cross-linking, or four-site cross-linking is lower than 10%.

3. The gel material according to claim 1 or 2, wherein the proportion of the imino residues in the gel obtained through the cross-linking in the three-site cross-linking or four-site cross-linking is lower than 15%; and further, the proportion of the imino residues in the gel obtained through the cross-linking in the three-site cross-linking or four-site cross-linking is lower than 10%.

4. The gel material according to claim 1 or 2, wherein cross-linking efficiency of a cross-linking reaction between the endogenous polyamine and the HA is higher than 75%; and
further, the efficiency of the cross-linking reaction between the endogenous polyamine and the HA is higher than 80%; and further, the efficiency of the cross-linking reaction between the endogenous polyamine and the HA is higher than 85%.

5. A method for preparing a gel material, comprising the following steps:

    regulating the pH of a mixed solution of HA and endogenous polyamine to 4.5-6.5, and adding an activating agent to cause the HA and the endogenous polyamine to undergo a two-site, three-site, or four-site cross-linking reaction, so as to obtain the gel material, wherein
    the endogenous polyamine comprises spermine and/or spermidine;
    when the activating agent comprises a carbonium salt and/or a phosphonium bromide salt formed by triphenylphosphine and bromide, the temperature of the cross-linking reaction is 10-60°C, and the time for the cross-linking reaction is 14-24 h; and
    when the activating agent comprises water-soluble carbodiimide and/or 4-(4,6-dimethoxytriazine-2-yl)-4-methylmorpholine hydrochloride, the temperature of the cross-linking reaction is 40-60°C, and the time for the cross-linking reaction is 14-24 h.

6. The method for preparing a gel material according to claim 5, wherein the pH of the mixed solution of the HA and the endogenous polyamine is regulated to 4.50-4.99 or 5.50-6.50; and the activating agent carbonium salt and/or phosphonium bromide salt formed by the triphenylphosphine and the bromide is added, the temperature of the cross-linking reaction being 10-60°C and the time for the cross-linking reaction being 14-24 h, to cause the HA and the endogenous polyamine to undergo the two-site cross-linking reaction, so as to obtain the gel material.

**7.** The method for preparing a gel material according to claim 5, wherein the pH of the mixed solution of the HA and the endogenous polyamine is regulated to 5.00-5.49; and the activating agent carbonium salt and/or phosphonium bromide salt formed by the triphenylphosphine and the bromide is added, the temperature of the cross-linking reaction being 10-60°C and the time for the cross-linking reaction being 14-24 h, to cause the HA and the endogenous polyamine to undergo the three-site or four-site cross-linking reaction, so as to obtain the gel material; or
the pH of the mixed solution of the HA and the endogenous polyamine is regulated to 5.00-5.49; and the activating agent water-soluble carbodiimide and/or 4-(4,6-dimethoxytriazine-2-yl)-4-methylmorpholine hydrochloride is added, the temperature of the cross-linking reaction being 40-60°C and the time for the cross-linking reaction being 14-24 h, to cause the HA and the endogenous polyamine to undergo the three-site or four-site cross-linking reaction, so as to obtain the gel material.

**8.** The preparation method according to any one of claims 5 to 7, wherein
the water-soluble carbodiimide comprises one or more of 1-ethyl-3-(3-dimethylaminopropyl)carbodiimide, 1-(3-dimethylaminopropyl)-3-ethyl-carbodiimide, 1-cyclohexyl-3-(2-morpholinoethyl)carbodiimide, 1,3-bis[di(methoxymethyl)methyl]carbodiimide, and salts thereof; the phosphonium bromide salt formed by the triphenylphosphine and the bromide comprises one or more of a phosphonium salt formed by the triphenylphosphine and carbon tetrabromide, and a phosphonium salt formed by the triphenylphosphine and N-bromosuccinimide; and the carbonium salt comprises one or more of O-(7-azabenzotriazole-1-yl)-bis(dimethylamino)carbonium hexafluorophosphate, O-(benzotriazol-1-yl)-bis(dimethylamino)carbonium hexafluorophosphate, O-(5-chlorobenzotriazol-1-yl)-bis(dimethylamino)carbonium hexafluorophosphate, O-(benzotriazol-1-yl)-bis(dimethylamino)carbonium tetrafluoroborate, O-(N-succinimidyl)-bis(d-imethylamino)carbonium tetrafluoroborate, and 2-(5-norborene-2,3-dicarboximido)-1,1,3,3-te-tramethyluronium tetrafluoroborate.

**9.** The preparation method according to any one of claims 5 to 7, wherein, when the water-soluble carbodiimide activating agent is used, the preparation method further comprises performing using in combination with an additive; and preferably, the additive comprises any one or more of N-hydroxysuccinimide, sulfonated N-hydroxysuccinimide, tert-butanol, and 1-hydroxybenzotriazole.

**10.** The preparation method according to any one of claims 5 to 7, wherein in the cross-linking reaction, during two-site cross-linking, an addition amount of the activating agent is 200-280% the amount of substance of the endogenous polyamine; and
during three-site and/or four-site cross-linking, the addition amount of the activating agent is 300-550% the amount of substance of the endogenous polyamine; and preferably, when the endogenous polyamine is the spermine, the addition amount of the activating agent is 400-550% the amount of substance of the spermine, and when the endogenous polyamine is the spermidine, the addition amount of the activating agent is 300-400% the amount of substance of the spermidine.

**11.** The preparation method according to any one of claims 5 to 7, wherein in the cross-linking reaction, when the endogenous polyamine is the spermine, an addition amount of the spermine is 0.3-35% of the mass of the HA; and when the endogenous polyamine is the spermidine, an addition amount of the spermidine is 0.5-40% of the mass of the HA;

further, a mass concentration of the HA in a mixed solution of a reaction system is 10-150 mg/mL; and
further, in finally-obtained cross-linked HA hydrogel, the mass concentration of the HA is 1-50 mg/mL.

**12.** The preparation method according to any one of claims 5 to 7, wherein the gel material is further treated through elution by an eluent, crushing, and drying;

preferably, the eluent is an organic solvent; more preferably, the organic solvent is soluble alcohol or soluble ketone; more preferably, the organic solvent is ethanol or acetone;
further, a volume ratio of the gel to the eluent in the reaction system during crushing is 1 :1-5; and
further, a particle size of the gel after crushing is 10-500 μm.

**13.** The preparation method according to claim 12, wherein the preparation method further comprising performing re-dissolution and moist heat sterilization on the dried gel material, wherein

preferably, a solution for the re-dissolution is a phosphate buffer solution; more preferably, a mass concentration of a phosphate buffer salt in the phosphate buffer solution is 5-40 mg/mL; and

further, the temperature of the moist heat sterilization is 120-130°C, and the time for the moist heat sterilization is preferably 15-45 min.

**14.** An application of the gel material according to any one of claims 1 to 4 or the gel material prepared by the preparation method according to any one of claims 5 to 13 in preparation of tissue filling and repair materials or drug carriers.

Fig. 1

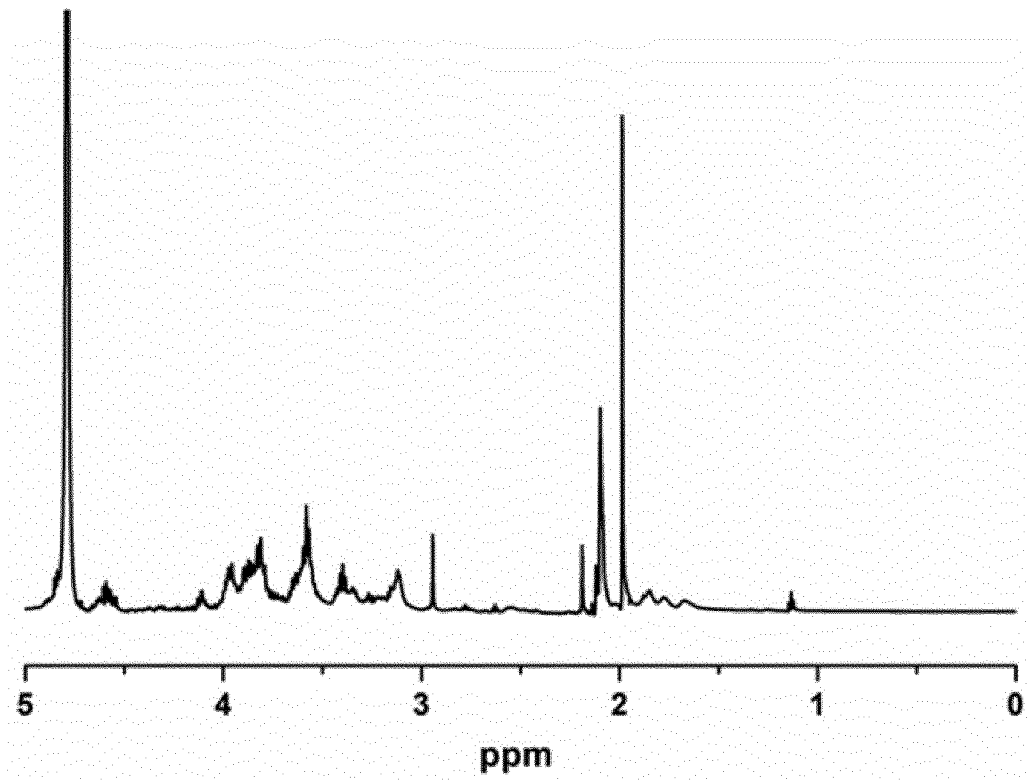
5
4
3
2
1
0
ppm

Fig. 2

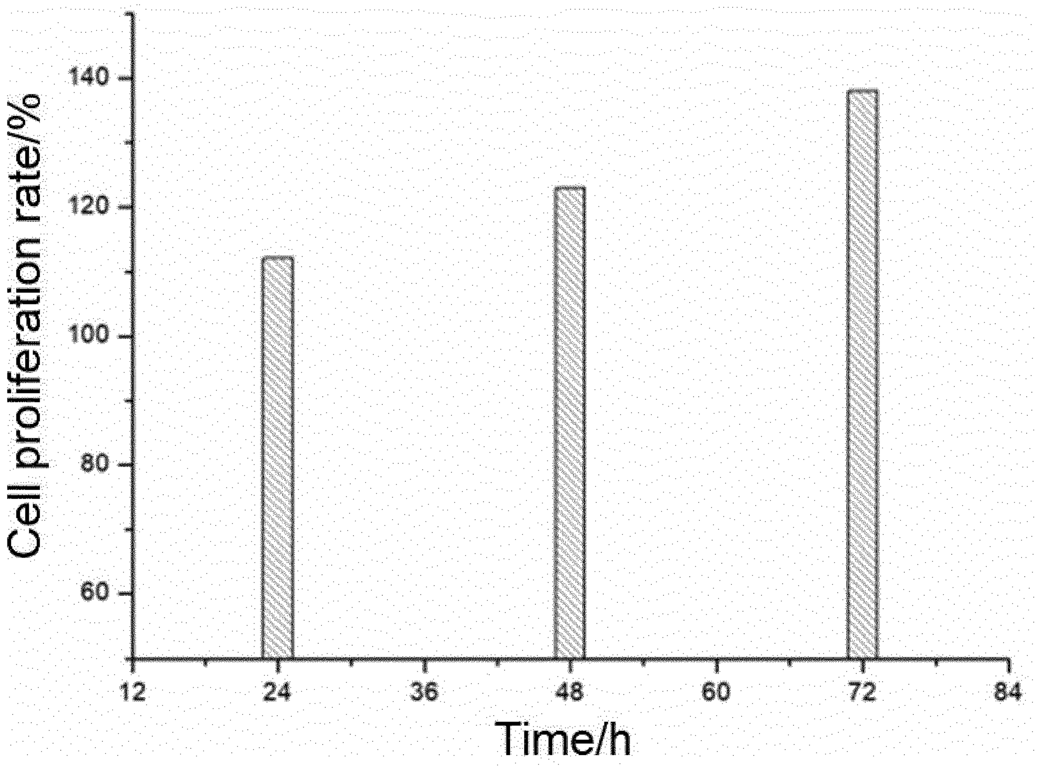
Cell proliferation rate/%
140
120
100
80
60
12
24
36
48
60
72
84
Time/h

Fig. 3

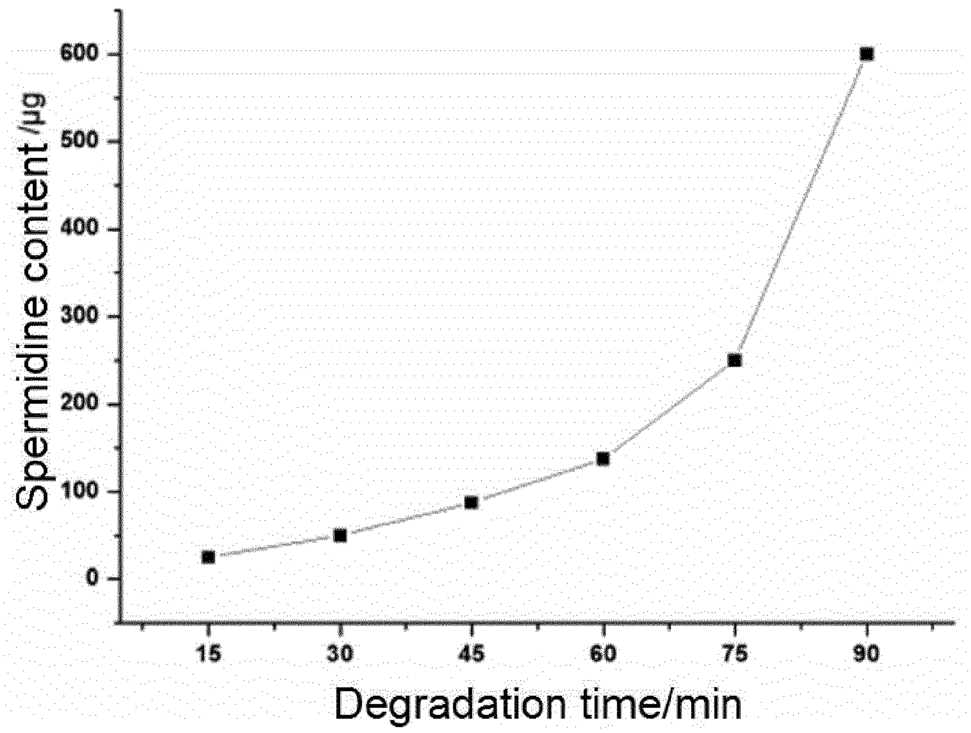
Spermidine content /μg
600
500
400
300
200
100
0
15
30
45
60
75
90
Degradation time/min

Fig. 4

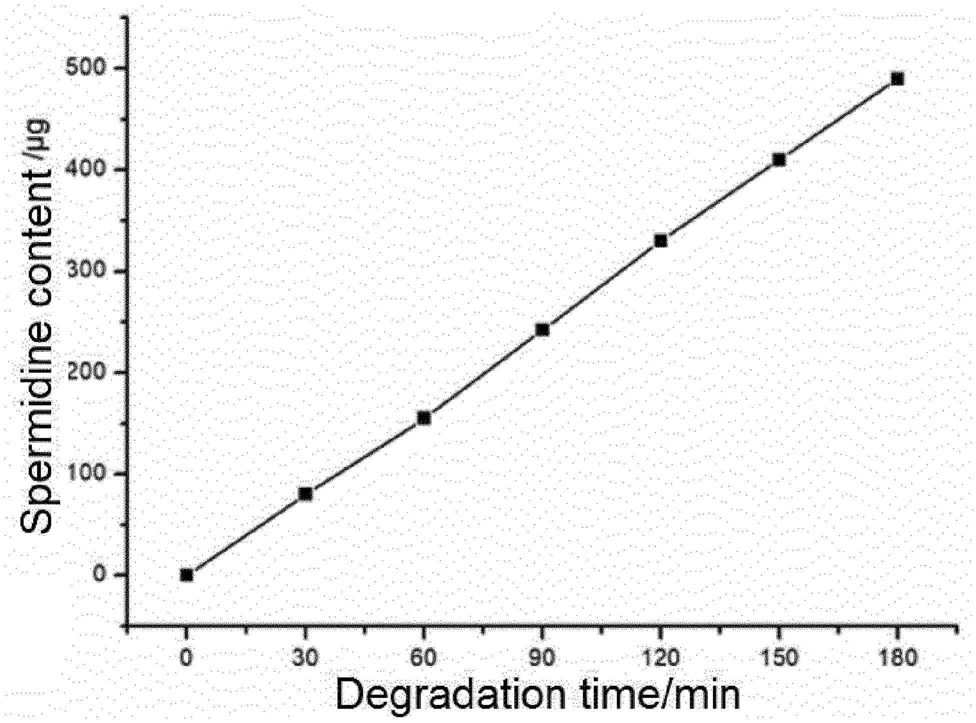
Spermidine content /μg
500
400
300
200
100
0
0
30
60
90
120
150
180
Degradation time/min

**INTERNATIONAL SEARCH REPORT**

International application No.
**PCT/CN2021/129743**

**A. CLASSIFICATION OF SUBJECT MATTER**

C08J 3/24(2006.01)i；C08J 3/075(2006.01)i；C08L 5/08(2006.01)i；C08K 5/17(2006.01)i；C08B 37/08(2006.01)i

According to International Patent Classification (IPC) or to both national classification and IPC

**B. FIELDS SEARCHED**

Minimum documentation searched (classification system followed by classification symbols)

C08J3/-,C08L5/-,C08K5/-,C08B37/-

Documentation searched other than minimum documentation to the extent that such documents are included in the fields searched

Electronic data base consulted during the international search (name of data base and, where practicable, search terms used)

CNPAT, CNKI, STN, WPI, EPODOC: 亚精胺, 透明质酸, 精胺, 凝胶, 玻尿酸, 精素, gel, hyaluronic, spermine, spermidine, hydrochloride, 71-44-3, 124-20-9, 9004-61-9

**C. DOCUMENTS CONSIDERED TO BE RELEVANT**

| Category* | Citation of document, with indication, where appropriate, of the relevant passages | Relevant to claim No. |
|---|---|---|
| X | WO 2014064632 A1 (TEOXANE) 01 May 2014 (2014-05-01)<br>description, embodiment 1, page 6, line 9-page 7, line 26, page 10, lines 9-29, page 11, line 31-page 12, line 21 | 1-14 |
| X | WO 2014064633 A1 (TEOXANE) 01 May 2014 (2014-05-01)<br>description, embodiments | 1-14 |
| A | CN 103189078 A (HUMEDIX CO., LTD.) 03 July 2013 (2013-07-03)<br>description, and abstract | 1-14 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **25 April 2022** | **28 July 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**
**Information on patent family members**

International application No.
**PCT/CN2021/129743**

| Patent document cited in search report | | | Publication date (day/month/year) | Patent family member(s) | | | Publication date (day/month/year) |
|---|---|---|---|---|---|---|---|
| WO | 2014064632 | A1 | 01 May 2014 | US | 2018140530 | A1 | 24 May 2018 |
| | | | | HU | E044362 | T2 | 28 October 2019 |
| | | | | CA | 2888616 | A1 | 01 May 2014 |
| | | | | HR | P20191196 | T1 | 18 October 2019 |
| | | | | KR | 20150079786 | A | 08 July 2015 |
| | | | | AU | 2013336274 | A1 | 07 May 2015 |
| | | | | JP | 2016507592 | A | 10 March 2016 |
| | | | | SI | 2912074 | T1 | 30 August 2019 |
| | | | | US | 2015272851 | A1 | 01 October 2015 |
| | | | | FR | 2997014 | A1 | 25 April 2014 |
| | | | | RS | 59079 | B1 | 30 September 2019 |
| | | | | PL | 2912074 | T3 | 30 September 2019 |
| | | | | DK | 2912074 | T3 | 22 July 2019 |
| | | | | RU | 2015118969 | A | 20 December 2016 |
| | | | | TR | 201911149 | T4 | 21 August 2019 |
| | | | | EP | 2912074 | A1 | 02 September 2015 |
| | | | | ES | 2735017 | T3 | 13 December 2019 |
| | | | | PT | 2912074 | T | 19 July 2019 |
| | | | | US | 9907739 | B2 | 06 March 2018 |
| | | | | FR | 2997014 | B1 | 20 March 2015 |
| | | | | AU | 2997014 | B1 | 24 August 2017 |
| | | | | BR | 112015009162 | A2 | 03 October 2017 |
| | | | | RU | 2653729 | C2 | 14 May 2018 |
| | | | | EP | 2912074 | B1 | 01 May 2019 |
| | | | | JP | 6521862 | B2 | 29 May 2019 |
| | | | | US | 10307362 | B2 | 04 June 2019 |
| | | | | KR | 102178252 | B1 | 13 November 2020 |
| WO | 2014064633 | A1 | 01 May 2014 | FR | 2997085 | A1 | 25 April 2014 |
| | | | | FR | 2997085 | B1 | 29 May 2015 |
| CN | 103189078 | A | 03 July 2013 | US | 2013252921 | A1 | 26 September 2013 |
| | | | | WO | 2012057381 | A1 | 03 May 2012 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 10105713211
- CN 106188609
- CN 106188584
- CN 111732741
- US 9907739 B2


### Non-patent literature cited in the description


- *Journal of Biomaterials Applications,* 2011, vol. 27 (2), 179-186
- **MADEO.** *Science,* 2018, vol. 359, 410